# EUROPEAN PATENT APPLICATION

(11) **EP 4 454 610 A1**
(43) Date of publication of application: **30.10.2024**
(21) Application number: 24167379.7
(22) Date of filing: 28.03.2024
(51) Int. Cl.: A61F 2/24, A61F 2/95

(54) **PROSTHETIC HEART VALVE DELIVERY SYSTEM HAVING AN IMPROVED COIL ASSEMBLY**

(30) Priority: 28.04.2023 US 202363462565 P; 28.02.2024 US 202418590260
(71) Applicant: Medtronic, Inc., Minneapolis, MN 55432 (US)
(72) Inventor: OWENSON, Caitlin M., Santa Rosa (US)
(74) Representative: Zimmermann & Partner Patentanwälte mbB

(57) **Abstract**

A delivery system for delivering a heart valve prosthesis includes a frame extending along a longitudinal axis between an inflow end and an outflow end. The frame includes a plurality of attachment members at one or more of the inflow end or the outflow end. The delivery system includes a coil assembly extending along a coil axis and attached to the plurality of attachment members. The coil assembly includes a first helical coil that receives a first attachment member of the plurality of attachment members. The coil assembly includes a second helical coil coaxial and intertwined with the first helical coil. The second helical coil receives a second attachment member of the plurality of attachment members. Methods of deploying a heart valve prosthesis at a treatment site are also provided.

## Description

### FIELD

The present disclosure relates generally to a prosthetic heart valve prostheses and, more particularly, to a delivery assembly or system for delivering a heart valve prosthesis.

### BACKGROUND

It is known to provide a delivery assembly or system for implanting a heart valve prosthesis within a target site of the vasculature of a patient. The heart valve prosthesis can be moved from a radially-collapsed position to a radially-expanded position. There is a continuing need to improve upon the devices available for heart valve replacement. In addition, there is a continuing need for improved transcatheter delivery systems and methods of implanting medical devices, and particularly systems related to heart valve replacement.

### SUMMARY

The following presents a simplified summary of the disclosure to provide a basic understanding of some aspects described in the detailed description.

In aspects, a delivery system for delivering a heart valve prosthesis including a frame and a prosthetic valve component including at least one leaflet attached to the frame. The frame extends along a longitudinal axis between an inflow end and an outflow end and includes a plurality of struts. The frame is configured to transition between a radially-collapsed position and a radially-expanded position. The frame includes a plurality of attachment members at one or more of the inflow end and the outflow end. The delivery system includes a coil assembly extending along a coil axis and configured to be releasably attached to the plurality of attachment members. The coil assembly includes a first helical coil wound around and extending along the coil axis and a second helical coil wound around and extending along the coil axis. The first helical coil terminates at a first helical end and is configured to receive a first attachment member of the plurality of attachment members. The second helical coil is coaxial and intertwined with the first helical coil. The second helical coil terminates at a second helical end that is axially aligned with the first helical end. The second helical coil is configured to receive a second attachment member of the plurality of attachment members.

In aspects, the first helical end and the second helical end are spaced circumferentially apart within a range from about 150 degrees to about 210 degrees.

In aspects, the first attachment member and the second attachment member are spaced circumferentially apart within a range from about 150 degrees to about 210 degrees. The first attachment member and the second attachment member are attached to the inflow end.

In aspects, the first helical coil is configured to receive a first number of attachment members of the plurality of attachment members and the second helical coil is configured to receive a second number of attachment members of the plurality of attachment members. The first number of attachment members is different than the second number of attachment members.

In aspects, the first number of attachment members are attached to a first circumferential half of the heart valve prosthesis and the second number of attachment members are attached to a second circumferential half of the valve prosthesis.

In aspects, a second coil assembly extends along a coil axis and is configured to be releasably attached to the plurality of attachment members. The second coil assembly includes a first helical coil wound around and extending along the coil axis and a second helical coil wound around and extends along the coil axis. The first helical coil terminates at a first helical end and is configured to receive a third attachment member of the plurality of attachment members. The second helical coil is coaxial and intertwined with the first helical coil. The second helical coil terminates at a second helical end that is axially aligned with the first helical end. The second helical coil is configured to receive a fourth attachment member of the plurality of attachment members.

In aspects, the third attachment member and the fourth attachment member are attached to the outflow end such that the second coil assembly is attached to the outflow end.

In aspects, a delivery system for delivering a heart valve prosthesis including a frame and a prosthetic valve component including at least one leaflet attached to the frame. The frame extends along a longitudinal axis between an inflow end and an outflow end and includes a plurality of struts. The frame is configured to transition between a radially-collapsed position and a radially-expanded position. The frame includes a plurality of attachment members at one or more of the inflow end and the outflow end. The delivery system includes a coil assembly extending along a coil axis and configured to be releasably attached to the plurality of attachment members. The delivery system includes a coil assembly extending along a coil axis and configured to be attached to the plurality of attachment members. The coil assembly includes a first helical coil, a second helical coil, and a third helical coil. The first helical coil is wound around and extends along the coil axis. The first helical coil terminates at a first helical end and is configured to receive a first attachment member of the plurality of attachment members. The second helical coil is wound around and extends along the coil axis. The second helical coil is coaxial and intertwined with the first helical coil. The second helical coil terminates at a second helical end that is axially aligned with the first helical end. The second helical coil is configured to receive a second attachment member of the plurality of attachment members. The third helical coil is wound around and extends along the coil axis. The third helical coil is coaxial and intertwined with the first helical coil and the second helical coil. The third helical coil terminates at a third helical end that is axially aligned with the first helical end and the second helical end. The third helical coil is configured to receive a third attachment member of the plurality of attachment members.

In aspects, the first helical end, the second helical end, and the third helical end are spaced apart within a range from about 90 degrees to about 150 degrees.

In aspects, the first attachment member, the second attachment member, and the third attachment member are spaced circumferentially apart within a range from about 90 degrees to about 150 degrees. The first attachment member, the second attachment member, and the third attachment member are attached to the inflow end.

In aspects, the first helical coil is configured to receive a first number of attachment members of the plurality of attachment members, the second helical coil is configured to receive a second number of attachment members of the plurality of attachment members, and the third helical coil is configured to receive a third number of attachment members of the plurality of attachment members. The first number of attachment members, the second number of attachment members, and the third number of attachment members are the same.

In aspects, a second coil assembly extends along a coil axis and is configured to be releasably attached to the plurality of attachment members. The second coil assembly includes a first helical coil, a second helical coil, and a third helical coil. The first helical coil is wound around and extends along the coil axis. The first helical coil terminates at a first helical end and is configured to receive a fourth attachment member of the plurality of attachment members. The second helical coil is wound around and extends along the coil axis. The second helical coil is coaxial and intertwined with the first helical coil. The second helical coil terminates at a second helical end that is axially aligned with the first helical end. The second helical coil is configured to receive a fifth attachment member of the plurality of attachment members. The third helical coil is wound around and extending along the coil axis. The third helical coil is coaxial and intertwined with the first helical coil and the second helical coil. The third helical coil terminates at a third helical end that is axially aligned with the first helical end and the second helical end. The third helical coil is configured to receive a sixth attachment member of the plurality of attachment members.

In aspects, the fourth attachment member, the fifth attachment member, and the sixth attachment member are attached to the outflow end.

In aspects, methods of deploying a heart valve prosthesis at a treatment site within a patient are provided. The heart valve prosthesis includes a frame and a prosthetic valve component including at least one leaflet attached to the frame. The frame extends along a longitudinal axis between an inflow end and an outflow end and includes a plurality of struts. The frame is configured to transition between a radially-collapsed position and a radially-expanded position. The frame includes a plurality of attachment members at one or more of the inflow end and the outflow end. The delivery system includes a coil assembly extending along a coil axis and configured to be releasably attached to the plurality of attachment members. The method includes receiving a first attachment member of the plurality of attachment members on a first helical coil. The first helical coil is wound around and extends along a coil axis. Methods include receiving a second attachment member of the plurality of attachment members on a second helical coil. The second helical coil is coaxial and intertwined with the first helical coil. Methods include percutaneously delivering the heart valve prosthesis in the radially-collapsed position to the treatment site. Methods include simultaneously rotating the first helical coil and the second helical coil to release the first attachment member from the first helical coil and the second attachment member from the second helical coil and cause the heart valve prosthesis to move from the radially-collapsed position to the radially-expanded position.

In aspects, the first attachment member and the second attachment member are released simultaneously.

In aspects, the first helical coil terminates at a first helical end and the second helical coil terminates at a second helical end. The first helical end and the second helical end are spaced circumferentially apart within a range from about 150 degrees to about 210 degrees.

In aspects, the first attachment member and the second attachment member are attached to the inflow end.

In aspects, receiving the first attachment member further includes receiving a first number of attachment members of the plurality of attachment members on the first helical coil. Receiving the second attachment member further includes receiving a second number of attachment members of the plurality of attachment members on the second helical coil. The first number of attachment members is different than the second number of attachment members.

In aspects, methods further include receiving a third attachment member of the plurality of attachment members on a third helical coil. The third helical coil is coaxial and intertwined with the first helical coil and the second helical coil.

In aspects, simultaneously rotating further includes simultaneously rotating the first helical coil, the second helical coil, and the third helical coil to release the first attachment member, the second attachment member, and the third attachment member.

In aspects, the frame of the heart valve prosthesis includes a plurality of sinusoidal rings, each ring being sutured to an adjacent ring via abutting crowns.

Further disclosed herein is a delivery system for delivering a heart valve prosthesis that includes a frame extending along a longitudinal axis between an inflow end and an outflow end, wherein the frame includes a plurality of attachment members at one or more of the inflow end or the outflow end, wherein the delivery system includes a coil assembly extending along a coil axis and attached to the plurality of attachment members, wherein the coil assembly includes a first helical coil that receives a first attachment member of the plurality of attachment members, wherein the coil assembly includes a second helical coil coaxial and intertwined with the first helical coil, wherein the second helical coil receives a second attachment member of the plurality of attachment members.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
FIG. 1 illustrates a perspective view of an exemplary transcatheter heart valve prosthesis in accordance with aspects of the disclosure;
FIG. 2 illustrates a side view of another exemplary transcatheter heart valve prosthesis in accordance with aspects of the disclosure;
FIG. 3 illustrates a side view of a delivery system for delivering the transcatheter heart valve prosthesis in accordance with aspects of the disclosure, wherein the delivery system includes a first coil assembly configured to engage an inflow end of a transcatheter heart valve prosthesis and a second coil assembly configured to engage an outflow end of the transcatheter heart valve prosthesis when the transcatheter heart valve prosthesis is loaded within the delivery system;
FIG. 4 is a cross-sectional view of the delivery system of FIG. 3 taken along line A-A of FIG. 3;
FIG. 5 illustrates an introducer sheath in accordance with aspects of the disclosure;
FIG. 6 illustrates an introducer sheath in accordance with aspects of the disclosure;
FIG. 7 schematically illustrates a side view of a transcatheter heart valve prosthesis and a pair of coil assemblies in accordance with aspects of the disclosure;
FIG. 8 illustrates a perspective view of an end of a coil assembly in accordance with aspects of the disclosure;
FIG. 9 illustrates an end view of the coil assembly in accordance with aspects of the disclosure;
FIG. 10 illustrates an end view of the coil assembly in which the coil assembly has been rotated in accordance with aspects of the disclosure;
FIG. 11 illustrates an end view of the coil assembly in which the coil assembly has been further rotated in accordance with aspects of the disclosure;
FIG. 12 illustrates an end view of the coil assembly in which the coil assembly has been further rotated in accordance with aspects of the disclosure;
FIG. 13 schematically illustrates a side view of a transcatheter heart valve prosthesis and a pair of coil assemblies in accordance with aspects of the disclosure;
FIG. 14 illustrates a perspective view of an end of a coil assembly in accordance with aspects of the disclosure;
FIG. 15 illustrates an end view of the coil assembly in accordance with aspects of the disclosure;
FIG. 16 illustrates an end view of the coil assembly in which the coil assembly has been further rotated in accordance with aspects of the disclosure;
FIG. 17 illustrates an end view of the coil assembly in which the coil assembly has been further rotated in accordance with aspects of the disclosure;
FIG. 18 illustrates an end view of the coil assembly in which the coil assembly has been further rotated in accordance with aspects of the disclosure;
FIG. 19 schematically illustrates a side view of a transcatheter heart valve prosthesis and a pair of coil assemblies with the transcatheter heart valve prosthesis in a radially-collapsed position in accordance with aspects of the disclosure;
FIG. 20 schematically illustrates a side view of the transcatheter heart valve prosthesis and the pair of coil assemblies after retracting a shaft in accordance with aspects of the disclosure;
FIG. 21 schematically illustrates a side view of a transcatheter heart valve prosthesis and a pair of coil assemblies with the transcatheter heart valve prosthesis in a radially-expanded position in accordance with aspects of the disclosure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. For example, a "substantially planar" surface is intended to denote a surface that is planar or approximately planar. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal" and "proximal" are used in the following description with respect to a position or direction relative to the treating clinician. "Distal" and "distally" are positions distant from or in a direction away from the clinician, and "proximal" and "proximally" are positions near or in a direction toward the clinician. In addition, the term "self-expanding" may be used in the following description with reference to one or more valve or stent structures of the prostheses hereof and is intended to convey that the structures are shaped or formed from a material that can be provided with a mechanical memory to return the structure from a compressed, collapsed, or constricted delivery configuration to an expanded deployed configuration or vice versa. Non-exhaustive exemplary self-expanding materials include stainless steel, a pseudo-elastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially collapsed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

Embodiments hereof relate to a delivery system for delivering of a heart valve prosthesis to a body lumen. The transcatheter heart valve prosthesis includes a plurality of first attachment members located at or near its inflow end, and a plurality of second attachment members located at or near its outflow end. The delivery system includes at least a first shaft and a second shaft, the first shaft being slidingly disposed over the second shaft. The delivery system also includes a retractable sheath slidingly disposed over the first shaft to hold the transcatheter heart valve prosthesis in a radially compressed configuration for delivery to a body lumen. A first coil assembly is disposed at a distal end of the first shaft, and a second coil assembly is disposed at a distal end of the second shaft. The second coil assembly of the second shaft is configured for engagement through openings in the plurality of second attachment members on the transcatheter heart valve prosthesis, and the first coil assembly of the first shaft is configured for engagement through openings in the plurality of first attachment members on the transcatheter heart valve prosthesis. The second coil assembly of the second shaft is configured to restrain the outflow end of the transcatheter heart valve prosthesis after the retractable sheath has been retracted to expose the outflow end of the transcatheter heart valve prosthesis *in situ,* and similarly the first coil assembly of the first shaft is configured to restrain the inflow end of the transcatheter heart valve prosthesis after the retractable sheath has been retracted to expose the inflow end of the transcatheter heart valve prosthesis *in situ.* The first and second coil assemblies provide for controlled release of the inflow and outflow ends, respectively, of the transcatheter heart valve prosthesis. More particularly, each coil assembly improves control and alignment during deployment and positioning of the transcatheter heart valve prosthesis, and each coil assembly can release the captured end of the transcatheter heart valve prosthesis at any time during deployment to suit any number of system characteristics driven by the therapy type, device type, or specific anatomical conditions that may prescribe the release timing. Typically, release of each coil assembly is activated after at least partial retraction of the retractable sheath, and thus provides a means of restraining the transcatheter heart valve prosthesis during positioning. Additional restraint of the transcatheter heart valve prosthesis is a key characteristic when the operator is attempting to accurately position the transcatheter heart valve prosthesis relative to an anatomical target. The second coil assembly of the second shaft in particular mitigates backfolding and/or infolding of the transcatheter heart valve prosthesis that may otherwise occur during deployment.

Each coil assembly includes a first helical coil wound around and extending along a coil axis and a second helical coil wound around and extending along the coil axis. The second helical coil is coaxial and intertwined with the first helical coil. The first helical coil terminates at a first helical end and the second helical coil terminates at a second helical end that is axially aligned with the first helical end. Each coil assembly is disposed over a shaft of the delivery system as described above, and the coaxial and intertwined coils collectively have a low profile to minimize the overall dimension of the delivery system. The first helical coil is configured to receive a first attachment member of the plurality of attachment members and the second helical coil is configured to receive a second attachment member of the plurality of attachment members. The first helical coil and the second helical coil are rotated concurrently and, due to the placement of the first and second attachment members on the first and second helical coils, respectively, the first and second attachment members are released simultaneously, for a more controlled release of the captured end of the prosthesis. The first and second attachments members that are released simultaneously may be positioned at circumferentially opposing positions of the transcatheter heart valve so that the release of the first and second attachment members is symmetric and circumferentially balanced.

The principles of the invention may be practiced in any instance in which it is desired to deliver a medical device intraluminally to a desired anatomic site. For the purpose of discussion, the invention will generally be described in the context in which the medical device being loaded onto a delivery system and delivered intraluminally is a prosthetic valve. In an embodiment, the prosthetic valve is configured to be implanted in the right ventricular outflow tract or the infundibulum, and corresponding embodiments of the invention are particularly useful for delivering the heart valve prosthesis to the right ventricular outflow tract. The transcatheter heart valve prosthesis may be used in anatomic locations other than the infundibulum, such as the right ventricular outflow tract and other locations in or near the heart. The purpose of such devices is to allow replacement or prosthetic valves, such as pericardial heart valves, for example, having a smaller diameter than the diameter of the implanted site (e.g., the right ventricular outflow tract) to be implanted. However, other uses of the invention, such as to deliver different medical devices to different locations in the body, are contemplated and are not limited to those discussed in the application.

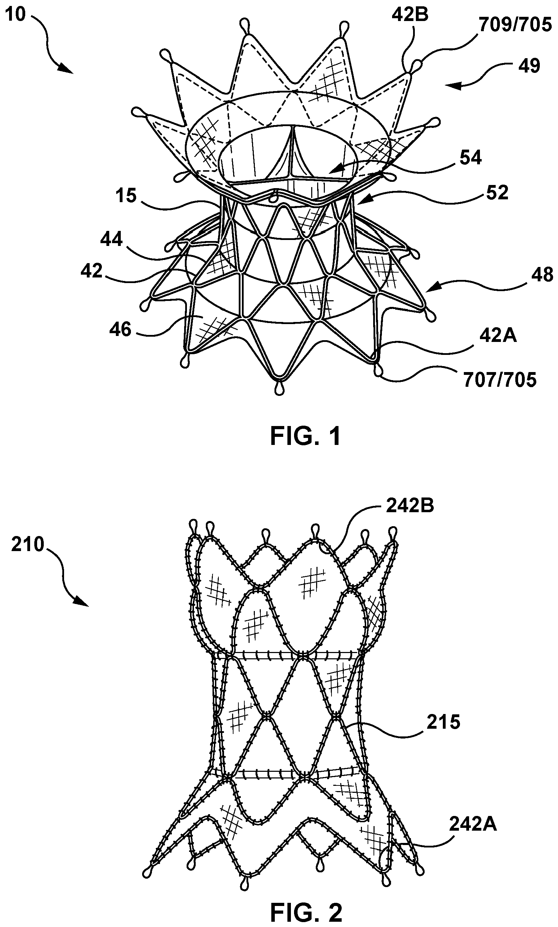

FIG. 1 is a perspective view of an exemplary transcatheter heart valve prosthesis 10 that may be deployed using embodiments of delivery systems described herein, while The transcatheter heart valve prosthesis 10 is merely exemplary. It is understood that any number of alternate devices can be used with the delivery devices and methods described herein. For example, U.S. Pub. No. 2022/0273437A1 to Kelley et al. and U.S. Patent No. 8,801,776 to House et al., each herein incorporated by reference in its entirety, describes a heart valve prosthesis that may be delivered by delivery systems described herein. In an embodiment, the transcatheter heart valve prosthesis 10 is the Medtronic HARMONY^{™} transcatheter pulmonary valve. In addition, the delivery systems described herein may also be used with other self-expanding prostheses such as other prosthetic heart valves, stent-graft prostheses, uncovered stents, bare metal stents, drug eluting stents, and any self-expanding structure. The transcatheter heart valve prosthesis 10 is illustrated to facilitate description of the disclosure. The following description of the transcatheter heart valve prosthesis 10 is merely exemplary in nature and is not intended to limit the invention or the application and uses of the invention.

The transcatheter heart valve prosthesis 10 includes a radially-expandable frame 15 and a prosthetic valve component 54. The frame 15 of the transcatheter heart valve prosthesis 10 supports the prosthetic valve component 54 within an interior of the frame 15. The frame 15 is configured to secure the prosthetic valve component 54 within the central lumen thereof and to secure the transcatheter heart valve prosthesis 10 in place in the vasculature of the patient. In the example shown in FIG. 1, the frame 15 is self-expanding or self-expandable to return to a radially expanded configuration from a radially compressed or constricted radially compressed configuration. However, this is not meant to be limiting, and the frame 15 can be balloon-expandable or mechanically expandable in other embodiments. In some embodiments, the transcatheter heart valve prosthesis 10 may be delivered to and implanted at a treatment site within a patient to replace any of an aortic valve, a pulmonic valve, a mitral valve, and a tricuspid valve. The valve to be replaced may be a native valve or a previously-implanted prosthetic valve, such as a failed surgical replacement valve or a failed transcatheter valve.

In the embodiment depicted in FIG. 1, the frame 15 has an expanded hourglass configuration including three longitudinal sections or portions of a relatively enlarged inflow end 48, a relatively enlarged outflow end 49, and a midportion 52 extending between the first and second ends 48, 49. The midportion 52 is generally cylindrical in shape with a smaller diameter than the inflow and outflow ends 48, 49. Each longitudinal section or portion of frame 15 may be designed with a number of different configurations and sizes to meet the different requirements of the location in which it may be implanted. Each longitudinal section or portion of the frame 15 may have the same or different cross-portion which may be for example circular, ellipsoidal, rectangular, hexagonal, rectangular, square, or other polygonal shape, although at present it is believed that circular or ellipsoidal may be preferable when the transcatheter heart valve prosthesis is being provided for replacement of the aortic or mitral valve. As alternatives to the deployed hourglass configuration of FIG. 1, the frame 15 may have an asymmetric hourglass configuration, a generally tubular configuration, or other stent configuration or shape known in the art for valve replacement.

One advantage of the midportion 52 having a smaller diameter than the inflow and outflow ends 48, 49 is to allow at least a portion of the midportion 52 of the frame 15 to hold or retain the prosthetic valve component 54 in its central lumen, when the prosthetic valve component 54 has a smaller diameter than the lumen in which the transcatheter heart valve prosthesis 10 is to be placed. The larger diameters of the inflow and outflow ends 48, 49 allow the transcatheter heart valve prosthesis 10 to be secured in place in a tubular organ, or a valved anatomic site, having a diameter larger than that of the prosthetic valve component 54 but smaller than the expanded diameter of the inflow and outflow ends 48, 49. The inflow and outflow ends 48, 49 are also shown to be flared, such that they gradually increase in diameter from where the inflow and outflow 48, 49 extend from the midportion 52. The angle at which the inflow and outflow ends 48, 49 are flared from the midportion 52 can vary depending on the desired maximum diameter and desired length of the frame 15.

The frame 15 includes a plurality of crowns 42 and a plurality of struts 44 with each crown 42 being formed between a pair of opposing struts 44. Each crown 42 is a curved segment or bend extending between opposing struts 44. A plurality of side openings are defined by the plurality of crowns 42 and the plurality of struts 44. A series of endmost inflow crowns 42A are formed at the inflow end 48 of the frame 15. The number of endmost inflow crowns 42A may vary according to size and application and may range, for example, between 6-15 crowns. In an embodiment, the inflow end 48 of the frame 15 has a total of nine endmost inflow crowns 42A. However, the configuration of the frame 15 is exemplary and other stent configurations are contemplated. For example, in another embodiment of a transcatheter heart valve prosthesis 210 as shown in FIG. 2, a frame 215 has a total of six endmost inflow crowns 242A. Similarly, a series of endmost outflow crowns 42B are formed at the outflow end 49 of the frame 15. The number of endmost outflow crowns 42B may vary according to size and application and may range, for example, between 6-15 crowns. In an embodiment, the outflow end 49 of the frame 15 has a total of nine endmost outflow crowns 42B. However, the configuration of the frame 15 is exemplary and other stent configurations are contemplated. For example, in the embodiment of FIG. 2, the frame 215 has a total of six endmost outflow crowns 242B.

In an embodiment, each of frames 15, 215 may be formed from a plurality of individual sinusoidal rings, sutured together at adjacent or abutting crowns in a "point-to-point" or "crown-to-crown" configuration as described in more detail in U.S. Pub. No. 2022/0273437A1 to Kelley et al., previously incorporated by reference. It will be understood by one of ordinary skill in the art that the sinusoidal rings at the inflow end of the frame 215 are not configured to be attached in a "point-to-point" or "crown-to-crown" configuration, as the two sinusoidal rings at the inflow end of the frame 215 are nested and have a gap therebetween.

As previously mentioned, the transcatheter heart valve prosthesis 10 includes the prosthetic valve component 54 positioned within the center lumen of the frame 15. The prosthetic valve component 54 includes at least one leaflet disposed within and secured to the frame 15. In the embodiment shown in FIG. 1, the prosthetic valve component 54 includes exactly three leaflets. However, this is not meant to be limiting, as the prosthetic valve component 54 may include more or fewer leaflets. The valve leaflets open and close to regulate flow through the transcatheter heart valve prosthesis 10. The prosthetic leaflets are attached to the frame 15 at commissures such that when pressure at the inflow end 48 exceeds pressure at the outflow end 49, the prosthetic leaflets open to allow blood flow through the heart valve prosthesis 10 from the inflow end 48 to the outflow end 49. When the pressure at the outflow end 49 exceeds pressure at the inflow end 48, the prosthetic leaflets close to prevent blood flow from the outflow end 49 to the inflow end 48.

The prosthetic valve component 54 is attached to (i.e., affixed to, held by, retained by, etc.) the frame 15 along its edges and is sutured or otherwise attached within the frame 15. The prosthetic valve component 54 included in the transcatheter heart valve prosthesis 10 may be a preserved bovine jugular vein of the type described in the above-cited Tower et al. references. Other vessels or donor species may, however, alternatively be employed. Alternatively, other substantially tubular valve bodies may be the prosthetic valve component 54 of the invention. For example, the prosthetic valve component 54 may be formed from a variety of materials including biological materials and polymers. Biological material includes homograft, allograft, or xenograft, with xenograft being common and well accepted and usually from bovine, ovine, swine or porcine pericardium, or a combination thereof. Polymers include expanded TEFLON^{™} polymers, high density polyethylene, polyurethane, and combinations thereof. Some examples of prosthetic valve components that may be used in the invention are described in U.S. Pat. Nos. 6,719,789 and 5,480,424, issued to Cox, which are incorporated herein by reference.

Graft material 46 encloses or lines the frame 15 as would be known to one of ordinary skill in the art of prosthetic tissue valve construction. The graft material 46 may be a natural or biological material such as pericardium or another membranous tissue such as intestinal submucosa. Alternatively, the graft material 46 may be a low-porosity woven fabric, such as polyester, Dacron fabric, or PTFE, which creates a one-way fluid passage when attached to the stent. In one embodiment, the graft material 46 may be a knit or woven polyester, such as a polyester or PTFE knit, which can be utilized when it is desired to provide a medium for tissue ingrowth and the ability for the fabric to stretch to conform to a curved surface, or may instead be ultra-high molecular weight polyethylene (UHMWPE), cotton, or the like. Polyester velour fabrics may alternatively be used, such as when it is desired to provide a medium for tissue ingrowth on one side and a smooth surface on the other side. These and other appropriate cardiovascular fabrics are commercially available from Bard Peripheral Vascular, Inc. of Tempe, Ariz., for example.

In aspects, the heart valve prosthesis 10 can comprise a plurality of attachment members 705 at one or both of the inflow end 48 and the outflow end 49. For example, the attachment members 705 can comprise a plurality of inflow end attachment members 707 attached to the inflow end 48, and a plurality of outflow end attachment members 709 attached to the outflow end 49. In aspects, the plurality of inflow end attachment members 707 may be attached to endmost inflow crowns 42A and the plurality of outflow end attachment members 709 may be attached to endmost outflow crowns 42B. In this way, the total number of attachment members may be dependent upon the design and configuration of the valve prosthesis 10, and, thus, there may be greater or fewer attachment members than as illustrated in FIG. 1.

Each attachment member of the attachment members 707, 709 defines an opening. The attachment members 707, 709 may comprise loops formed from sutures and may be formed from the material used to form part of the prosthetic valve component 54 or other materials. In aspects, the attachment members 707, 709 can be formed from an ultra-high-molecular-weight polyethylene ("UHMWPE") thread, for example, since this material comprises properties of being durable and lubricous, as well as hydrophobic, which can help to minimize swelling or clotting due to contact with blood. Other materials may be used, however, that may comprise some or all of these attachment properties. Other attachment members 707, 709 besides loops are also contemplated, and such attachment members may be configured to connect, fasten, or attach the heart valve prosthesis 10 to a delivery system, allowing for the collapse (e.g., radially-collapsed) of the heart valve prosthesis 10 for insertion into the vasculature of a patient. The attachment members 707, 709 can selectively disengage or disconnect from the delivery system to release the valve prosthesis 10 at a desired treatment site, as described in more detail herein.

While the attachment members 707, 709 are illustrated as being attached to each endmost inflow crown 42A of the inflow end 48 and each endmost crown 42B of the outflow end 49, such a location of the attachment members 707, 709 is not intended to be limiting. Rather, in aspects, not all of the endmost crowns may be attached to the delivery system 300 during delivery and, thus, not all of the endmost crowns are required to include an attachment member attached thereto. Further, in aspects, some or all of the endmost crowns may comprise an attachment member but not all attachment members are required to be loaded onto the delivery system.

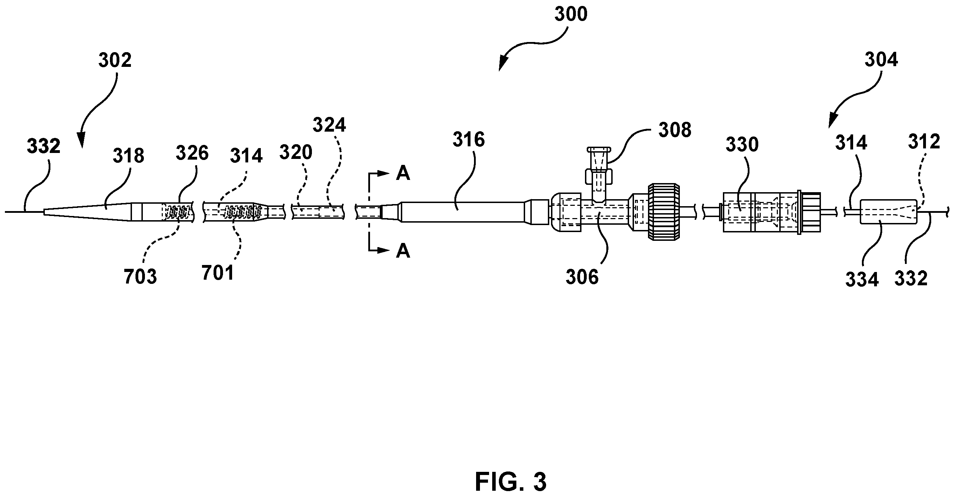

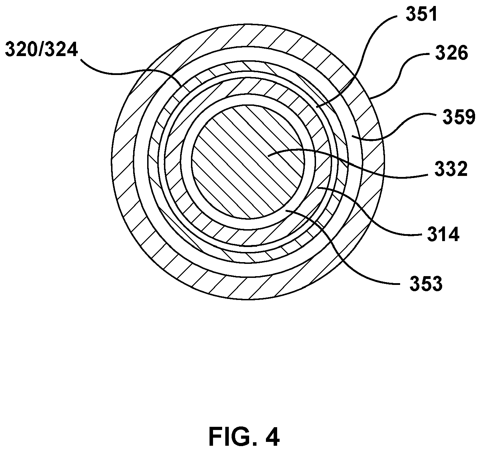

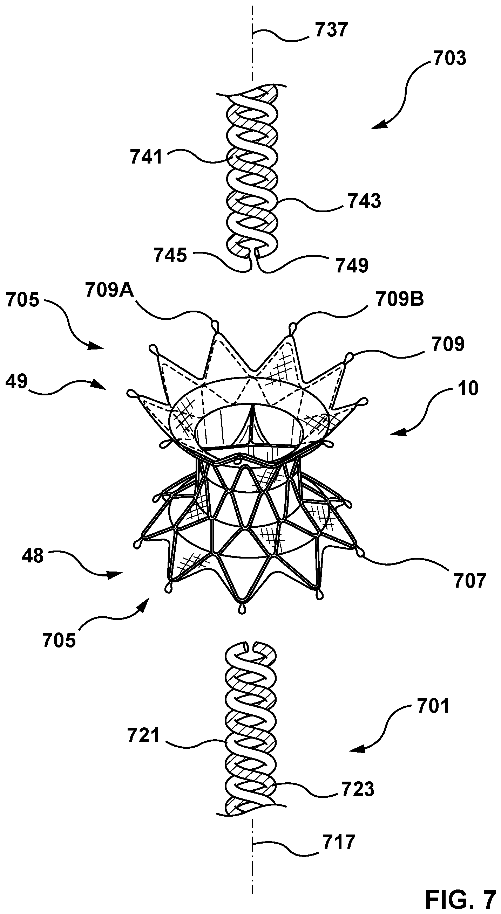

FIG. 3 shows a side view of a delivery system 300 for delivering and deploying a transcatheter heart valve prosthesis (e.g., transcatheter heart valve prosthesis 10) according to embodiments hereof. One skilled in the art will realize that FIGS. 3 and 4 illustrate one example of a delivery system and that components illustrated in FIGS. 3 and 4 may be removed and/or additional components may be added. The delivery system 300 is configured to receive the transcatheter heart valve prosthesis 10 and percutaneously deliver the transcatheter heart valve prosthesis 10 in a compressed or radially compressed configuration to a treatment site *in situ.* The delivery system 300 can comprise one or more coil assemblies, for example, a first coil assembly 701 and a second coil assembly 703. More particularly, the delivery system 300 includes a first coil assembly 701 configured to engage or attach to the plurality of inflow end attachment members 707 on or near the inflow end 48 of the transcatheter heart valve prosthesis 10 and a second coil assembly 703 configured to engage or attach to the plurality of outflow end attachment members 709 on or near the outflow end 49 of the transcatheter heart valve prosthesis 10. As will be described in more detail herein, the second coil assembly 703 is configured for engagement through openings in the plurality of outflow end attachment members 709 that is rotatable to release the plurality of outflow end attachment members 709 and the first coil assembly 701 is configured for engagement through openings in the plurality of inflow end attachment members 707 that is rotatable to release the plurality of inflow end attachment members 707. The structure and function of the first and second coil assemblies are described in more detail herein with respect to FIGS. 7-12.

FIG. 3 illustrates the delivery system 300 disposed over a guidewire 332 for illustrative purposes only. With any of the systems and methods described herein, the guidewire 332 may be initially introduced into the target treatment site through a suitable body lumen to properly locate the desired position for the transcatheter heart valve prosthesis 10. The remainder of the delivery system 300 may then be guided along the guidewire 332 and into the site. The guidewire 332 may be, for example a 0.089 cm extra stiff guidewire as manufactured by Amplatzer, Golden Valley, Minn., U.S.A.

The delivery system 300 includes a distal end generally designated by the reference numeral 302 and a proximal end generally designated by the reference number 304. The proximal end 304 of the delivery system 300 remains outside of the patient, and the distal end 302 is inserted into the patient and is delivered intravascularly to an area at or near a pulmonary valve inside the body. Other uses for the delivery system 300 in other areas of the body, however, are also contemplated. The proximal end 304 includes means for remotely controlling the distal end 302 of the delivery system 300, in particular relating to deploying or releasing the transcatheter heart valve prosthesis 10 from the delivery system 300 *in situ.*

The components of the proximal end 304 of the delivery system 300 may include those shown in FIG. 3, although additional and/or alternative components are also contemplated. The proximal end 304 includes a first rotating homeostasis valve 306, a side access port 308, a second rotating homeostasis valve 330, and a handle 334 including a guidewire lumen inlet 312. The first rotating homeostasis valve 306 grips or forms a fluid seal around an outer surface of the delivery system 300 to prevent blood or other fluid from leaking out of the delivery device at the proximal end or entry site into a patient and is configured to allow wires, devices and fluid to pass through. Furthermore, the first rotating homeostasis valve 306 also controls the components of the distal end as described in more detail below by rotation of a portion of the first rotating homeostasis valve 306. The side access port 308 is provided as a means for injecting contrast media or saline, for example, into the delivery system 300. The second rotating homeostasis valve 330 also prevents blood or other fluid from leaking back through the delivery system 300 and is configured to allow wires, devices, and fluid to pass through. Furthermore, the second rotating homeostasis valve 330 also controls the components of the distal end as described in more detail below by rotation of a portion of the second rotating homeostasis valve 330. The first and second valves rotating homeostasis valve 306, 330 are exemplary. Other alternative or additional components of the proximal end 304 of the delivery system 300 are also contemplated by the invention.

FIG. 3 shows the distal end 302 of the delivery system 300 in an assembled configuration, without a transcatheter heart valve prosthesis attached or loaded. FIG. 4 is a cross-sectional view of the delivery system of FIG. 3 taken along line A-A of FIG. 3. As best shown on FIG. 4, the delivery system 300 includes at least three concentric tubular components. More particularly, the delivery system 300 includes a first or intermediate shaft 320 which defines a lumen 353, a second or inner shaft 314 which defines a guidewire lumen 351, and a retractable sheath 326 which defines a lumen 359. The first or intermediate shaft 320 is slidingly disposed over the second or inner shaft 314. The retractable sheath 326 is slidingly disposed over the first or intermediate shaft 320 to hold the transcatheter heart valve prosthesis 10 in a radially collapsed or compressed configuration during delivery. The retractable sheath 326 is made of a low friction and flexible material, such as polytetrafluoroethylene (PTFE), polyurethane, silicone, or polyethylene is retractable via a retraction mechanism such as a knob of the first rotating homeostasis valve 306. Various other retraction mechanisms may be used, such as an axially-slidable lever, a rotatable rack and pinion gear, or other known mechanisms. In this way, the retractable sheath 326 is retractable relative to the inner and outer shafts 314, 320 during deployment of the transcatheter heart valve prosthesis 10. The delivery system 300 may further include an outer sleeve 316 that is disposed over a proximal portion of the retractable sheath 326. The purpose of the sleeve 316 is to keep blood from leaking back around the delivery system 300.

A tapered distal tip 318 is attached to the inner shaft 314 and serves to ease the passage of the delivery system 300 through the vasculature. The inner shaft 314 extends along the entire length of the delivery system 300 and is configured to be tracked over the guidewire 332. The inner shaft 314 may be formed of one or more polymeric materials, non-exhaustive examples of which include polyethylene, polyethylene block amide copolymer (PEBA), polyamide and/or combinations thereof, either laminated, blended or co-extruded. Optionally, the inner shaft 314 may be formed as a composite having a reinforcement layer incorporated within a polymeric body in order to enhance strength and/or torque-ability. Suitable reinforcement layers include braiding, wire mesh layers, embedded axial wires, embedded helical or circumferential wires, hypotubes, and the like. The inner shaft 314 is attached to the handle 334 at its proximal end. The handle 334 includes the guidewire lumen inlet 312 such that the handle 334 and the inner shaft 314 may be tracked over the guidewire 332. Inner shaft 314 may be independently advanced or retracted through other components of the delivery system 300 by moving the handle 334 to which it is attached. In addition, the inner shaft 314 may be independently rotated relative to the other components of the delivery system 300 by rotating or turning the handle 334 to which it is attached. In an embodiment, the handle 334 may include an actuator (not shown) such as a knob that is operatively coupled to the inner shaft 314 such that rotation of the actuator results in rotation of the inner shaft 314.

In this embodiment, the second coil assembly 703 is coupled to a distal end of the second or inner shaft 314. More particularly, the second coil assembly 703 is attached to a proximal end of the distal tip 318, and the distal tip 318 is attached or secured to the distal end of the inner shaft 314. When the inner shaft 314 is rotated by rotating or turning the handle 334 to which it is attached, the inner shaft 314, the distal tip 318 and the second coil assembly 703 collectively rotate as an assembly. Thus, movement of the inner shaft 314, as controlled by the handle 334, controls movement or rotation of the second coil assembly 703.

The intermediate shaft 320 is slidingly disposed over the inner shaft 314. The intermediate shaft 320 may be formed of one or more polymeric materials, non-exhaustive examples of which include polyethylene, polyethylene block amide copolymer (PEBA), polyamide and/or combinations thereof, either laminated, blended or co-extruded. Surrounding the intermediate shaft 320 is a reinforcement layer 324, which is attached or otherwise bonded to the intermediate shaft 320 and serves to reinforce the intermediate shaft 320. Optionally, the intermediate shaft 320 may be formed as a composite having a reinforcement layer incorporated within a polymeric body in order to enhance strength and/or torque-ability. Suitable reinforcement layers include braiding, wire mesh layers, embedded axial wires, embedded helical or circumferential wires, hypotubes, and the like. Movement of the intermediate shaft 320 is controlled by the second rotating homeostasis valve 330 at the proximal end 304 of the delivery system 300. A portion of the second rotating homeostasis valve 330 is rotated or otherwise manipulated in order to rotate the intermediate shaft 320 or move the intermediate shaft 320 proximally and distally as desired. The first coil assembly 701 is coupled to a distal end of the first or intermediate shaft 320. When the intermediate shaft 320 is rotated by rotating or turning a portion of the second rotating homeostasis valve 330, the intermediate shaft 320 and the first coil assembly 701 collectively rotate as an assembly. Thus, movement of the intermediate shaft 320, as controlled by the second rotating homeostasis valve 330, controls movement or rotation of the first coil assembly 701.

The first and second coil assemblies 701, 703 provides means for releasably attaching the transcatheter heart valve prosthesis 10 onto the delivery system 300 by holding the inflow and outflow ends 48, 49, respectively, of the transcatheter heart valve prosthesis 10 on the delivery system 300 during delivery. The first coil assembly 701 is configured to hold the inflow end 48 of the transcatheter heart valve prosthesis 10 by engaging through openings in the plurality of inflow end attachment members 707, while the second coil assembly 703 is configured to hold the outflow end 49 of the transcatheter heart valve prosthesis 10 by engaging through openings in the plurality of outflow end attachment members 709. The first and second coil assemblies 701, 703 are further configured to release the transcatheter heart valve prosthesis 10 from the delivery system 300 *in situ.* The inner shaft 314 is rotatable in order to release the plurality of outflow end attachment members 709. Stated another way, when the second coil assembly 703 rotates or turns via rotation of the inner shaft 314, the plurality of outflow end attachment members 709 disengage or detach from the second coil assembly 703, thus releasing the outflow end 49 of the transcatheter heart valve prosthesis 10. Similarly, the intermediate shaft 320 is rotatable in order to release the plurality of inflow end attachment members 707. Stated another way, when the first coil assembly 701 rotates or turns via rotation of the intermediate shaft 320, the plurality of inflow end attachment members 707 disengage or detach from the first coil assembly 701, thus releasing the inflow end 48 of the transcatheter heart valve prosthesis 10. The speed that the inflow and outflow ends 48, 49 of the transcatheter heart valve prosthesis 10 are released from the delivery system 300 are controlled by the rate of rotation of the first and second coil assemblies 701, 703, respectively, therefore preventing uncontrolled release of the transcatheter heart valve prosthesis 10. The inflow and outflow ends 48, 49 of the transcatheter heart valve prosthesis 10 are selectively held, restrained, or otherwise controlled by the first and second coil assemblies 701, 703, respectively, until the accurate positioning of the transcatheter heart valve prosthesis 10 is established. The coils of the first and second coil assemblies 701, 703 have opposite winding directions such that the plurality of inflow end attachment members 707 coupled to the first coil assembly 701 move in a distal direction along the coil winding(s) to release the inflow end 48 of the transcatheter heart valve prosthesis 10 while the plurality of outflow end attachment members 709 coupled to the second coil assembly 703 move in a proximal direction along the coil winding(s) to release the outflow end 49 of the transcatheter heart valve prosthesis 10.

Although the delivery system 300 is depicted with the three elongated tubular shaft components with the second coil assembly 703 being coupled to the inner shaft 314, this is not meant to be limiting. As described in U.S. Pub. No. 2021/0346158A1 to Orth et al., assigned to the same assignee as the present disclosure and herein incorporated by reference in its entirety, another delivery system for use with embodiments of the first and second coil assemblies described herein may include four elongated tubular shaft components with the second coil assembly 703 being coupled to a second intermediate shaft that is disposed between the inner shaft 314 and the (first) intermediate shaft 320. The second intermediate shaft, and the second coil assembly 703 attached thereto, is rotatable relative to the inner shaft 314 in order to release the outflow end attachment members 709 from the second coil assembly 703 and the inner shaft 314 (and the distal tip 318 attached thereto) is not required to rotate during the deployment of the transcatheter heart valve prosthesis.

Minimally invasive percutaneous interventional procedures, including endovascular procedures, require access to the venous or arterial system. In general, it is desirable to make the smallest incision point with the shortest tissue contact time when entering the body. Small incisions and short tissue contact time generally lead to improved patient outcomes, less complications, and less trauma to the vessels or organs being accessed, as well as less trauma to the skin and tissue through which the access point is created. Access is required for various medical procedures that deliver or implant structural elements (such as heart valves, heart valve repair devices, occluders, grafts, electrical stimulators, leads, etc.) percutaneously. Some procedures employ relatively large devices that require relatively large sheaths to deliver the devices to the intended site within the body. With such procedures, access site trauma can occur, often resulting in vessel damage, excessive bleeding, increased case time, increased risk of infection, and increased hospitalization time. To reduce access trauma, physicians try to use the smallest devices possible and place the smallest sheath size. This can be problematic, however, if during the procedure the physician discovers a larger device is needed. This leads to a need to upsize the sheath, which is a lengthy procedure and leads to increased risk to the patient. Expandable sheaths can be expanded within the body and thus do not require removal to upsize.

Expandable sheath designs may be regionally or locally expansive to selectively and temporarily expand when the device is passing through a region of the sheath and to retract or recover when the device is not passing or has already passed through the sheath. Embodiments disclosed herein may be employed with an expandable introducer sheath that may solve these and other issues that contribute to vascular trauma. The expandable introducer sheath disclosed herein is described with respect to percutaneous access for transcatheter heart valve repair or replacement, and it should be understood that one or more features of the expandable introducer sheath may be employed alone or in combination for other medical procedures requiring percutaneous access, including but not limited to placement of stents, angioplasty, removal of arterial or venous calcification, and pre-dilatation or post-dilatation.

Various embodiments disclosed herein may include an introducer sheath that has a selectively expandable diameter to allow for the passage of a relatively larger device therethrough and further is configured to return to its original diameter upon passage of the device. The various embodiments may reduce damage to surrounding tissues by reducing contact with those tissues and by eliminating the need to exchange sheaths of different sizes. As a result, in comparison to known sheaths, these embodiments can reduce procedure time, vascular trauma, bleeding, and the resulting risk of infection and other complications. However, it should be understood that the present disclosure is not limited for use with an expandable introducer sheath. Rather, one or more features of the present disclosure can be employed either alone or in combination without an introducer sheath, with a non-expandable introducer sheath, or with an expandable introducer sheath. Likewise, if employed, the introducer sheath may be an integrated introducer sheath (e.g., an introducer sheath integrated with a delivery system) or a non-integrated introducer sheath (e.g., an introducer sheath separate from the delivery system but provided for use with the delivery system).

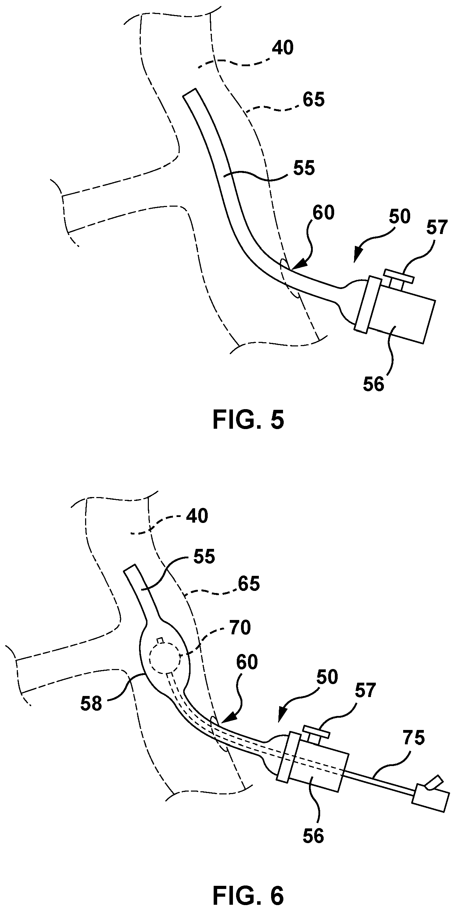

FIGS. 5 and 6 depict one embodiment of an introducer sheath 50 positioned through an incision 60 in the skin 65 of a patient and into a vessel 40 of a patient. The sheath 50 has a tubular shaft 55 and a proximal hub 56 with a hemostatic seal and a luer lock 57. FIG. 5 shows the sheath 50 positioned in the vessel 40 in its normal, unexpanded state, while FIG. 6 shows the sheath 50 positioned in the vessel 40 with a delivery device 75 delivering another device 70 that is being advanced through the sheath 50 such that the tubular shaft 55 expands or deforms at the location where the device 70 is passing through. The shaft 55 expands at expanded region 58 when the device 70 passes through and then retracts or recovers to its original diameter after the device 70 moves past or is removed from the shaft 55. Thus, the tubular shaft 55 is configured to be expandable and retractable.

In certain embodiments, the expandability of the shaft 55 (and any shaft described according to any embodiment set forth herein) is achieved via the elasticity of the shaft 55, which can result in the shaft 55 being either self-expandable or self-expanding or mechanically expandable or mechanically expanding. For purposes of this application, self-expandable means that the shaft 55 is configured to expand to a predetermined or nominal diameter automatically (without any type of actuation, mechanical or otherwise). Further, for purposes of this application, mechanically expandable means that the shaft 55 is configured to expand when a positionable medical device is positioned through the shaft 55. That is, the device itself that is being passed through the shaft 55 causes the expansion of the shaft 55, as depicted in FIG. 6. Alternatively, the expandable characteristics of the shaft 55 can be caused by something other than elasticity.

After passage of the device, the shaft 55 is configured to be contractable, retractable, or recoverable to its original, unexpanded state as depicted in FIG. 5. The retractability can be, in certain embodiments, achieved by the elasticity of the shaft 55, which can result in the shaft 55 being either self-retractable or self-retracting, self-recoverable, or self-contractable, or mechanically retractable or mechanically retracting, mechanically recoverable, or mechanically contractable. For purposes of this application, self-retractable means that the shaft 55 is configured to retract to a predetermined or nominal diameter automatically (without any type of actuation, mechanical or otherwise). Further, for purposes of this application, mechanically retractable means that the shaft 55 is configured to retract when a device or component is used to cause the shaft 55 to retract or recover. Alternatively, the retractable characteristics of the shaft 55 can be caused by something other than elasticity.

For purposes of this application, any device that can be positioned through an introducer sheath according to any embodiment disclosed or contemplated herein can be referred to as a positionable medical device or insertable medical device. Such devices include guidewires, dilators, delivery devices (for delivery and/or placement of structural elements such as heart valves, heart valve repair devices, occluders, grafts, electrical stimulators, leads, etc.), guide catheters, guiding sheaths, diagnostic catheters, stent delivery systems, balloon catheters, and other known vascular devices. Other devices can include non-vascular devices such as scopes and other common surgical instruments. Further, the introducer sheath is configured to receive tissues or organs. Thus, as one non-limiting example, the introducer sheath 50 is described as being an expandable introducer sheath 50 for introduction of the delivery system 300 including the transcatheter heart valve prosthesis 10.

Turning now to FIG. 7, the structure and function of the first and second coil assemblies 701, 703 will be described in more detail. In FIG. 7, the first and second coil assemblies 701, 703 are shown removed from the delivery system 300 for sake of clarity and illustration only.

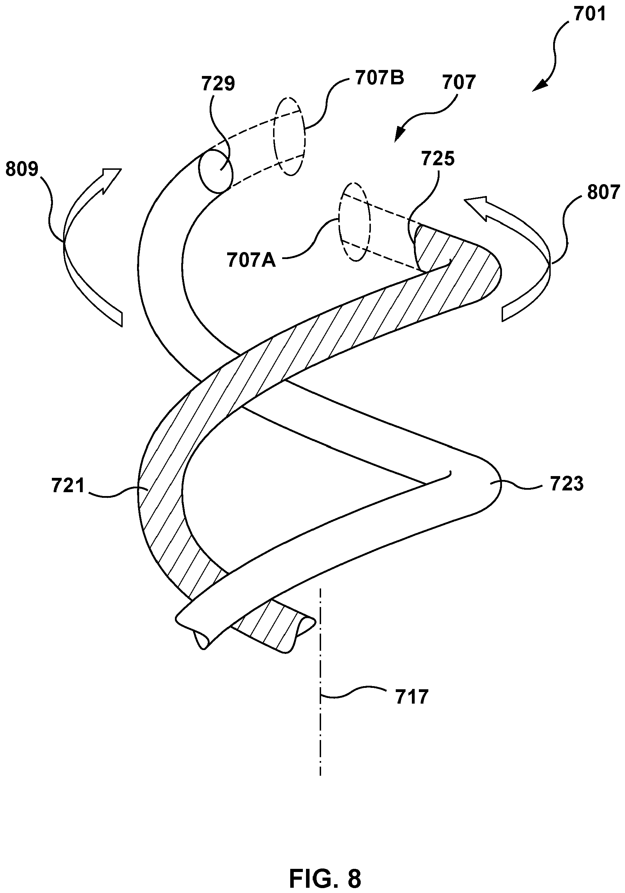

Referring first to the first coil assembly 701, the first coil assembly 701 extends along a coil axis 717 and is configured to be releasably attached to the plurality of attachment members 705, for example, the inflow end attachment members 707. The first coil assembly 701 comprises a first helical coil 721 and a second helical coil 723. The first helical coil 721 can be wound around and extend along the coil axis 717, and may comprise a corkscrew or spiral staircase shape that is a smooth space curve with tangent lines at a constant angle to a fixed axis. For example, by being wound around, the first helical coil 721 can extend circumferentially around the coil axis 717, with the first helical coil 721 comprising a substantially constant radial separating distance from the coil axis 717 (e.g., or substantially constant diameter) along the coil axis 717. The first helical coil 721 can terminate at a first helical end 725 (e.g., illustrated in FIG. 8).

The second helical coil 723 can be wound around and extend along the coil axis 717, and may comprise a corkscrew or spiral staircase shape that is a smooth space curve with tangent lines at a constant angle to a fixed axis. For example, by being wound around, the second helical coil 723 can extend circumferentially around the coil axis 717, with the second helical coil 723 comprising a substantially constant radial separating distance from the coil axis 717 (e.g., or substantially constant diameter) along the coil axis 717. The second helical coil 723 can terminate at a second helical end 729 (e.g., illustrated in FIG. 8). The second helical end 729 can be axially aligned with the first helical end 725, such that the first helical end 725 and the second helical end 729 may be located at substantially the same axial location along the coil axis 717. The first helical coil 721 and the second helical coil 723 can be substantially identical in structure, size, and shape, with the first helical coil 721 and the second helical coil 723 intertwined. For example, along a surface of the first coil assembly 701 parallel to the coil axis 717, the first helical coil 721 and the second helical coil 723 can alternate positions, with the first helical coil 721 and the second helical coil 723 comprising substantially identical shapes but differing by a translation along the coil axis 717. As used herein, "intertwined" includes that the windings of the first helical coil 721 are disposed between the windings of the second helical coil 723 such that, except at the ends of the first coil assembly 701, each winding of the first helical coil 721 is disposed between a pair of windings of the second helical coil 723 and each winding of the second helical coil 723 is disposed between a pair of windings of the first helical coil 721. The first helical coil 721 and the second helical coil 723 are both disposed over the intermediate shaft 320 of the delivery system 300, and coupled thereto such that the first and second helical coils 721, 723 rotate or turn as an assembly with rotation of the intermediate shaft 320.

The second coil assembly 703 is substantially identical in structure, shape, and function to the first coil assembly 701. More particularly, the second coil assembly 703 extends along a coil axis 737 (e.g., substantially identical to the coil axis 715) and is configured to be releasably attached to the plurality of attachment members 705, for example, the outflow end attachment members 709. The second coil assembly 703 can comprise a first helical coil 741 (e.g., substantially identical to the first helical coil 721) and a second helical coil 743 (e.g., substantially identical to the second helical coil 723). The first helical coil 741 can be wound around and extend along the coil axis 737, and may comprise a corkscrew or spiral staircase shape that is a smooth space curve with tangent lines at a constant angle to a fixed axis. For example, by being wound around, the first helical coil 741 can extend circumferentially around the coil axis 737, with the first helical coil 741 comprising a substantially constant radial separating distance from the coil axis 737 (e.g., or substantially constant diameter) along the coil axis 737. The first helical coil 741 can terminate at a first helical end 745.

The second helical coil 743 can be wound around and extend along the coil axis 737, and may comprise a corkscrew or spiral staircase shape that is a smooth space curve with tangent lines at a constant angle to a fixed axis. For example, by being wound around, the second helical coil 743 can extend circumferentially around the coil axis 737, with the second helical coil 743 comprising a substantially constant radial separating distance from the coil axis 737 (e.g., or substantially constant diameter) along the coil axis 737. The second helical coil 743 can terminate at a second helical end 749. The second helical end 749 can be axially aligned with the first helical end 745, such that the first helical end 745 and the second helical end 749 may be located at substantially the same axial location along the coil axis 737. The first helical coil 741 and the second helical coil 743 can be substantially identical in structure, size, and shape, with the first helical coil 741 and the second helical coil 743 intertwined. For example, along a surface of the second coil assembly 703 parallel to the coil axis 737, the first helical coil 741 and the second helical coil 743 can alternate positions, with the first helical coil 741 and the second helical coil 743 comprising substantially identical shapes but differing by a translation along the coil axis 737. As used herein, "intertwined" includes that the windings of the first helical coil 741 are disposed between the windings of the second helical coil 743 such that, except at the ends of the second coil assembly 703, each winding of the first helical coil 741 is disposed between a pair of windings of the second helical coil 743 and each winding of the second helical coil 743 is disposed between a pair of windings of the first helical coil 741. The first helical coil 741 and the second helical coil 743 are both disposed over the inner shaft 314 of the delivery system 300, and coupled thereto such that the first and second helical coils 741, 743 rotate or turn as an assembly with rotation of the inner shaft 314.

FIG. 8 illustrates an end of the first coil assembly 701 comprising the first helical end 725 of the first helical coil 721 and the second helical end 729 of the second helical coil 723. FIG. 8 illustrates two attachment members for the purposes of illustration and to more clearly show a possible position of the attachment members relative to the helical ends 725, 729. However, in operation, additional attachment members can be provided at the inflow end 48 and the outflow end 49, similar to the valve prosthesis 10 of FIG. 7. As illustrated in FIG. 8, the inflow end attachment members 707 can comprise a first attachment member 707A and a second attachment member 707B. The first helical coil 721 can receive the first attachment member 707A and the second helical coil 723 can receive the second attachment member 707B. For example, the attachment members 707A, 707B can comprise an opening or an eyelet that is sized such that the helical coils 721, 723 can pass therethrough. For example, the first attachment member 707A can be received on the first helical coil 721 and the second attachment member 707B can be received on the second helical coil. In this way, methods can comprise receiving the first attachment member 707A of the inflow end attachment members 707 on the first helical coil 721, and receiving the second attachment member 707B of the inflow end attachment members 707 on the second helical coil 723.

To attach the attachment members 707A, 707B to the helical coils 721, 723, the helical coils 721, 723 can be positioned adjacent to the helical ends 725, 729, for example, with the first attachment member 707A positioned adjacent to the first helical end 725 and the second attachment member 707B positioned adjacent to the second helical end 729. Next, the first coil assembly 701 can be rotated in a first rotational direction 807 and/or the valve prosthesis 10 can be rotated in a second rotational direction 809 that is opposite the first rotational direction 807. In this way, rotating the first coil assembly 701 in the first rotational direction 807 and/or the valve prosthesis 10 in the second rotational direction 809 can cause the helical ends 725, 729 to move closer to the attachment members 707A, 707B, thus allowing the attachment members 707A, 707B to be received onto the helical coils 721, 723. The first coil assembly 701 and/or the valve prosthesis 10 can continue to be rotated in the rotational directions 807, 809, until some or all of the inflow end attachment members 707 are received by one of the first helical coil 721 and the second helical coil 723.

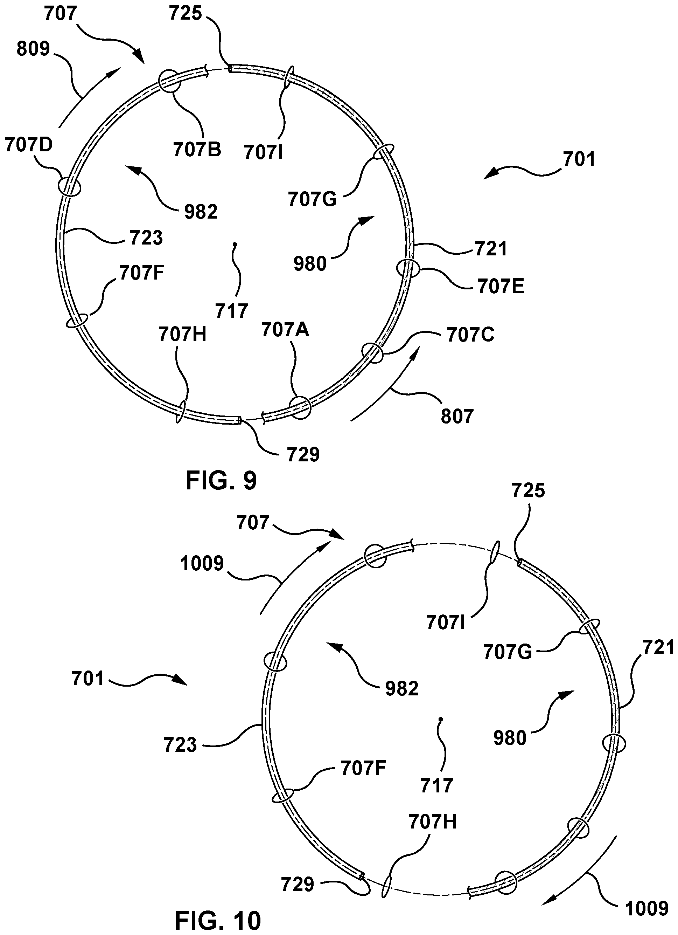

FIG. 9 illustrates an end view of the first coil assembly 701, in which the inflow end attachment members 707 are received on one of the first helical coil 721 or the second helical coil 723. In the depicted embodiment, the transcatheter heart valve prosthesis includes a total of nine inflow end attachment members 707. For sake of illustration, the nine inflow end attachment members 707 are referred to herein as first attachment member 707A, second attachment member 707B, third attachment member 707C, fourth attachment member 707D, fifth attachment member 707E, sixth attachment member 707F, seventh attachment member 707G, eighth attachment member 707H, and ninth attachment member 7071.

In the depicted embodiment, the first helical coil 721 can receive a first number 980 of attachment members of the inflow end attachment members 707, and the second helical coil 723 receiving a second number 982 of attachment members of the inflow end attachment members 707. In aspects, the first number 980 of attachment members may be different than the second number 982 of attachment members. For example, in FIG. 9, the first number 980 of attachment members comprises five attachment members (namely attachment members 707A, 707C, 707E, 707G, 707I), and the second number 982 of attachment members comprises four attachment members (namely attachment members 707B, 707D 707F, 707H), such that the first number 980 may be greater than the second number 982. However, in other aspects, the numbers 980, 982 of attachment members may be the same, or the second number 982 may be greater than the first number 980. In aspects, the first number 980 of attachment members may be attached to a first circumferential half of the valve prosthesis 10 and the second number 982 of attachment members may be attached to an opposing second circumferential half of the valve prosthesis 10. The first circumferential half can span about 180 degrees of the inflow end 48 of the valve prosthesis 10 to which the first number 980 of attachment members are attached, and the second circumferential half can span the remaining 180 degrees of the inflow end 48 of the valve prosthesis 10 to which the second number 982 of attachment members are attached.

As illustrated in FIG. 9, the first helical end 725 and the second helical end 729 are spaced circumferentially apart (e.g., about the coil axis 717) within a range from about 150 degrees to about 210 degrees, or about 180 degrees. As illustrated in FIGS. 7-8, the first helical end 725 and the second helical end 729 may be located at substantially the same axial location along the coil axis 717, such that a plane that is perpendicular to the coil axis 717 can intersect the first helical end 725 and the second helical end 729. The first helical coil 721 can receive the ninth attachment member 707I of the inflow end attachment members 707 and the second helical coil 723 can receive the eighth attachment member 707H of the inflow end attachment members 707. In aspects, the ninth attachment member 7071 and the eighth attachment member 707H may be spaced circumferentially apart within a range from about 150 degrees to about 210 degrees, or about 180 degrees, with the ninth attachment member 7071 and the eighth attachment member 707H attached to the inflow end 48 (e.g., illustrated in FIG. 7) of the valve prosthesis 10. The seventh attachment member 707G can be spaced circumferentially apart from the ninth attachment member 707I and received on the first helical coil 721, while the sixth attachment member 707F can be spaced circumferentially apart from the eighth attachment member 707H and received on the second helical coil 723.

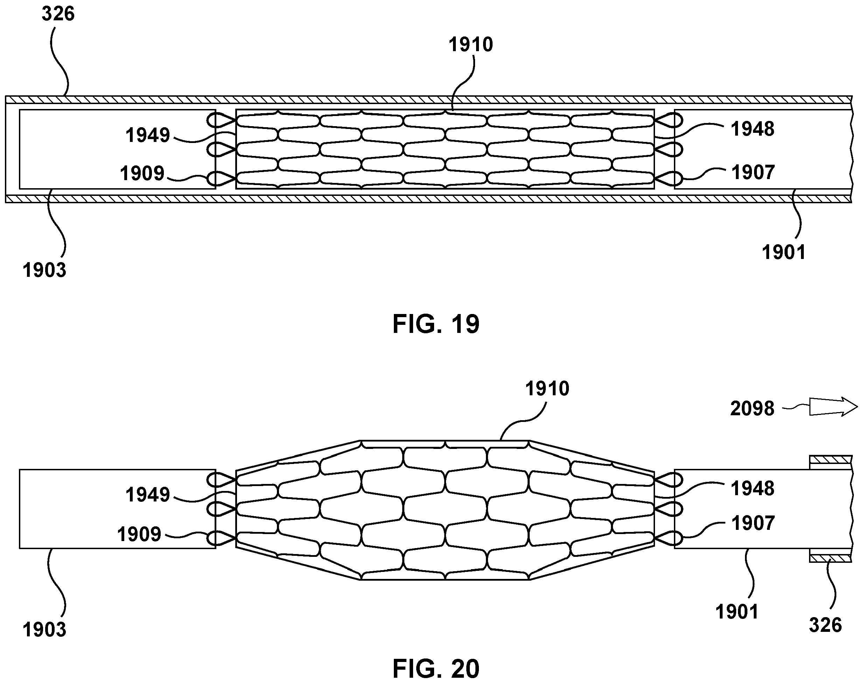

FIG. 9 illustrates the first coil assembly 701 after the first coil assembly 701 has been rotated in the first rotational direction 807 and/or the valve prosthesis 10 has been rotated in the second rotational direction 809 (e.g., also illustrated in FIG. 8) to receive the inflow end attachment members 707. In this way, the final attachment member to be received by the first helical coil 721 is the ninth attachment member 707I and the final attachment member to be received by the second helical coil 723 is the eighth attachment member 707H. Accordingly, in this way, methods can comprise receiving the first number 980 of attachment members on the first helical coil 721, and receiving the second number 982 of attachment members on the second helical coil 723. After the first coil assembly 701 receives the inflow end attachment members 707, and, likewise, after the second coil assembly 703 receives the outflow end attachment members 709, the valve prosthesis 10 may be in the radially-collapsed position (e.g., illustrated in FIGS. 19-20).

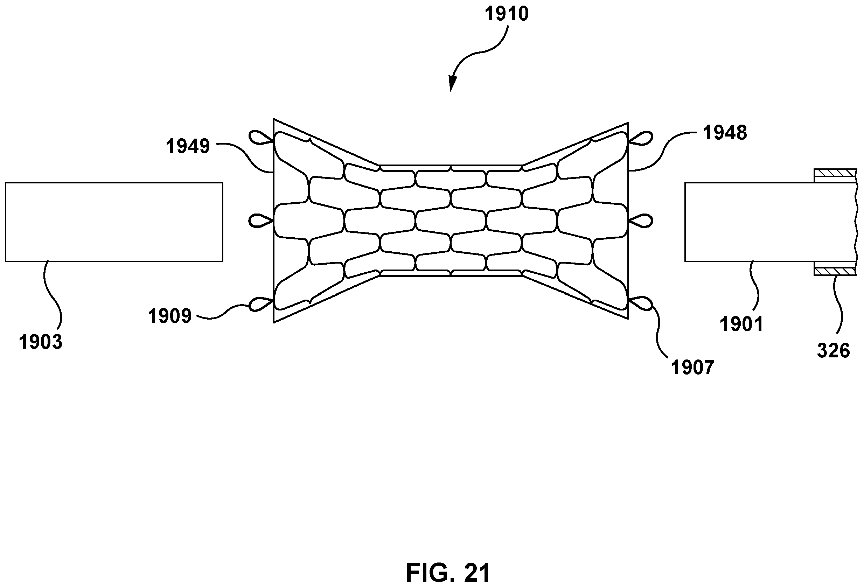

FIG. 10 illustrates the first coil assembly 701 after the first coil assembly 701 has been rotated in a second rotational direction 1009 that is opposite the first rotational direction 807. For example, the first helical coil 721 and the second helical coil 723 can be rotated in the second rotational direction 1009. In aspects, the first rotational direction 807 is opposite the second rotational direction 1009 such that if the first rotational direction 807 is counter-clockwise, the second rotational direction 1009 is clockwise. By rotating the first coil assembly 701 in the second rotational direction 1009, the first coil assembly 701 can release at least some of the inflow end attachment members 707. For example, methods comprise simultaneously rotating the first helical coil 721 and the second helical coil 723 to release the ninth attachment member 707I from the first helical coil 721 and the eighth attachment member 707H from the second helical coil 723 to facilitate movement of the heart valve prosthesis 10 from the radially-collapsed position (e.g., illustrated in FIGS. 19-20) to the radially-expanded position (e.g., illustrated in FIG. 7 and FIG. 21).

In aspects, the ninth attachment member 707I and the eighth attachment member 707H can be released simultaneously. For example, due to the ninth attachment member 707I and the eighth attachment member 707H being spaced circumferentially apart about 180 degrees, due to the helical ends 725, 729 being spaced circumferentially apart about 180 degrees, and due to the first helical coil 721 and the second helical coil 723 being rotated simultaneously, the ninth attachment member 707I and the eighth attachment member 707H can be removed from the helical coils 721, 723 at substantially the same time. The first helical end 725 may pass through the ninth attachment member 707I at substantially the same time that the second helical end 729 passes through the eighth attachment member 707H.

Due to the structure of the intertwined first and second helical coils 721, 723, the helical ends 725, 729 are circumferentially separated from each other, with a gap or space therebetween as best shown on FIG. 8. This gap between the helical ends 725, 729 ensures that the ninth attachment member 707I and the eighth attachment member 707H do not interfere with each other when being released from the first coil assembly 701. In addition, this gap between the helical ends 725, 729 causes the ninth attachment member 707I and the eighth attachment member 707H to be released with circumferentially opposing positions in the anatomy. Stated another way, the ninth attachment member 707I and the eighth attachment member 707H are released from the helical ends 725, 729 with optimal placement in the anatomy, adjacent to the final intended deployment site within the anatomy. Lastly, this gap between the helical ends 725, 729 allows for easier loading of the attachment members as described with respect to FIG. 8.

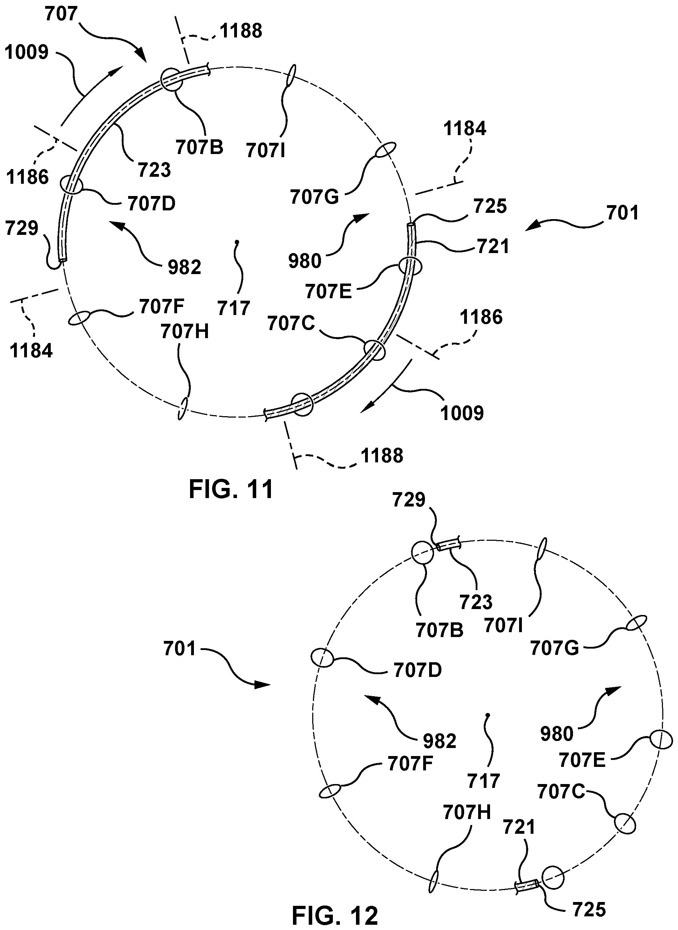

FIG. 11 illustrates the first coil assembly 701 continuing to rotate in the second rotational direction 1009 following the position in FIG. 10. As the first coil assembly 701 rotates, additional attachment members can be released. For example, the first helical coil 721 can be further rotated to release seventh attachment member 707G and the second helical coil 723 can be further rotated to release the sixth attachment member 707F. In aspects, the seventh attachment member 707G and the sixth attachment member 707F can be released simultaneously due to the first helical end 725 exiting the seventh attachment member 707G at substantially the same time that the second helical end 729 exits the sixth attachment member 707F and due to a circumferential spacing between the attachment members 707G, 707F substantially matching the circumferential spacing between the helical ends 725, 729.

FIG. 11 illustrates a plurality of rotational positions 1184, 1186, 1188 (e.g., illustrated schematically with dashed lines to show where the helical ends 725, 729 would be at the various rotational positions 1184, 1186, 1188) at which the first coil assembly 701 can release the inflow end attachment members 707. For example, when the first helical end 725 and the second helical end 729 reach a first rotational position 1184, the seventh attachment member 707G and the sixth attachment member 707F have been released. When the first helical end 725 and the second helical end 729 reach a second rotational position 1186, another pair of attachment members 707E, 707D (e.g., which are spaced apart circumferentially about 180 degrees) have been simultaneously released. The first coil assembly 701 can continue to rotate such that when the first helical end 725 and the second helical end 729 reach a third rotational position 1188, another pair of attachment members 707C, 707B (e.g., which are spaced apart circumferentially about 180 degrees) have been simultaneously released. Accordingly, in this way, as the first coil assembly 701 rotates in the second rotational direction 1009, the first helical coil 721 and the second helical coil 723 can simultaneously release pairs (e.g., 707I and 707H, 707G and 707F, 707E and 707D, 707C and 707B) of attachment members. In aspects, if the number of attachment members on the first helical coil 721 differs from the number of attachment members on the second helical coil 723, then one attachment member (i.e., attachment member 707A) may be released alone (e.g., not as a pair). As illustrated in FIG. 12, the first coil assembly 701 can continue to rotate in the second rotational direction 1009 at least until all of the inflow end attachment members 707 have been released. In aspects, after releasing the inflow end attachment members 707, the inflow end 48 of the valve prosthesis 10 can move from the radially-collapsed position to the radially-expanded position.

FIGS. 8-12 illustrate an exemplary process of the loading and release of the inflow end attachment members 707 by the first coil assembly 701. In aspects, the loading and release of the outflow end attachment members 709 can occur in a substantially identical manner by the second coil assembly 703. For example, the second coil assembly 703 comprises the first helical coil 741 and the second helical coil 743, wherein the helical coils 741, 743 of the second coil assembly 703 are substantially identical to the helical coils 721, 723 of the first coil assembly 701. The helical coils 741, 743 can terminate at the helical ends 745, 749 which are substantially identical to the helical ends 725, 729 of the helical coils 721, 723. The first helical coil 741 can receive a first attachment member 709A of the outflow end attachment members 709 and the second helical coil 743 can receive a second attachment member 709B of the outflow end attachment members 709. The second coil assembly 703 can be rotated in a first rotational direction (e.g., similar to the rotation of the first coil assembly 701 in the rotational direction 807) to receive the remaining attachment members of the outflow end attachment members 709 and, thus, causing the outflow end 49 of the valve prosthesis 10 to radially-compress. The second coil assembly 703 can then be rotated in an opposing second rotational direction (e.g., similar to the rotation of the first coil assembly 701 in the rotational direction 1009) to release the outflow end attachment members 709 and, thus, allow the outflow end 49 of the valve prosthesis 10 to radially-expand.

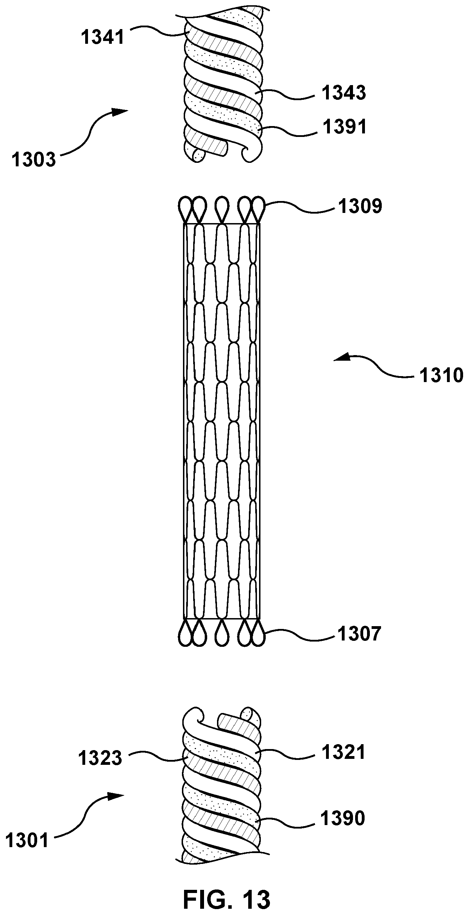

Turning now to FIGS. 13-18, another embodiment of coil assemblies that may be utilized with the delivery system 300 are shown. In FIG. 13, a first coil assembly 1301 and a second coil assembly 1303 are shown removed from the delivery system 300 for sake of clarity and illustration only. Each of the first and second coil assemblies 1301, 1303 include three coils rather than two coils as shown in the first and second coil assemblies 701, 703. A coil assembly with three coils is configured to simultaneously release a trio of attachment members rather than a pair of attachment members as in the previous embodiment of a coil assembly with two coils. A transcatheter heart valve prosthesis 1310 is shown with a plurality of inflow end attachment members 1307 and a plurality of outflow end attachment members 1309. The transcatheter heart valve prosthesis 1310 may be similar to the transcatheter heart valve prostheses 10, 210 described above.

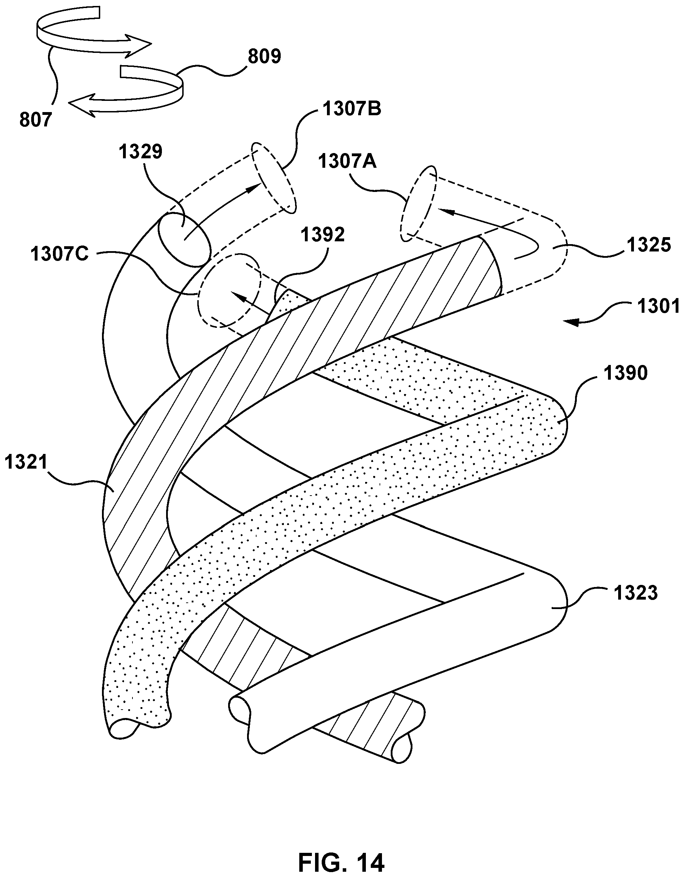

The first coil assembly 1301 comprises a first helical coil 1321, a second helical coil 1323, and a third helical coil 1390. The first helical coil 1321 and the second helical coil 1323 of the first coil assembly 1301 are substantially similar to the first helical coil 721 and the second helical coil 723 of the first coil assembly 701 illustrated in FIGS. 7-12. The third helical coil 1390 can be wound around, and extend along, the coil axis 1317. The third helical coil 1390 can be intertwined with the first helical coil 1321 and the second helical coil 1323, with the third helical coil 1390 comprising substantially the same shape as the first helical coil 1321 and the second helical coil 1323, for example, by comprising a corkscrew or spiral staircase shape that is a smooth space curve with tangent lines at a constant angle to a fixed axis. The third helical coil 1390 can terminate at a third helical end 1392 (e.g., illustrated in FIG. 14) that is axially aligned with a first helical end 1325 of the first coil 1321 and a second helical end 1329 of the second coil 1323.

The second coil assembly 1303 can comprise a first helical coil 1341, the second helical coil 1343, and a third helical coil 1391. The first helical coil 1341 and the second helical coil 1343 of the second coil assembly 1303 may be substantially identical to the first helical coil 741 and the second helical coil 743 of the first coil assembly 1301 illustrated in FIGS. 7-12. The third helical coil 1391 can be wound around, and extend along, a coil axis 737. The third helical coil 1391 can be intertwined with the first helical coil 1341 and the second helical coil 1343, with the third helical coil 1391 comprising substantially the same shape as the first helical coil 1341 and the second helical coil 1343, for example, by comprising a corkscrew or spiral staircase shape that is a smooth space curve with tangent lines at a constant angle to a fixed axis. The third helical coil 1391 can terminate at a third helical end 1393 that is axially aligned with a first helical end 1345 of the first coil 1341 and a second helical end 1349 of the second coil 1343. In aspects, the second coil assembly 1303 can be substantially identical in structure, shape, and function to the first coil assembly 1301.

FIG. 14 illustrates an end of the first coil assembly 1301 comprising the first helical end 1325 of the first helical coil 1321, the second helical end 1329 of the second helical coil 1323, and the third helical end 1392 of the third helical coil 1390. FIG. 14 illustrates three attachment members for the purposes of illustration and to more clearly show a possible position of the attachment members relative to the helical ends 1325, 1329, 1392. However, in operation, additional attachment members can be provided at the inflow end 48 and the outflow end 49, similar to the valve prosthesis 1310 of FIG. 13. Methods can comprise receiving a first attachment member 1307A on the first helical coil 1321, receiving a second attachment member 1307B on the second helical coil 1323, and receiving a third attachment member 1307C on the third helical coil 1390. The attachment members 1307A, 1307B, 1307C can be attached to the helical coils 1321, 1323, 1390 in substantially the same manner as described relative to FIG. 8. For example, the first attachment member 1307A can be positioned adjacent to the first helical end 1325, the second attachment member 1307B can be positioned adjacent to the second helical end 1329, and the third attachment member 1307C can be positioned adjacent to the third helical end 1392. Next, the first coil assembly 1301 can be rotated in the first rotational direction 807 and/or the valve prosthesis 1310 can be rotated in the second rotational direction 809 that is opposite the first rotational direction 807. In this way, rotating the first coil assembly 1301 in the first rotational direction 807 and/or the valve prosthesis 1310 in the second rotational direction 809 can cause the helical ends 1325, 1329, 1392 to move closer to the attachment members 1307A, 1307B, 1307C, thus allowing the attachment members 1307A, 1307B, 1307C to be received onto the helical coils 1321, 1323, 1390. The first coil assembly 1301 and/or the valve prosthesis 1310 can continue to be rotated in the rotational directions 807, 809, until some or all of the inflow end attachment members 1307 are received by one of the first helical coil 1321, the second helical coil 1323, and the third helical coil 1390.

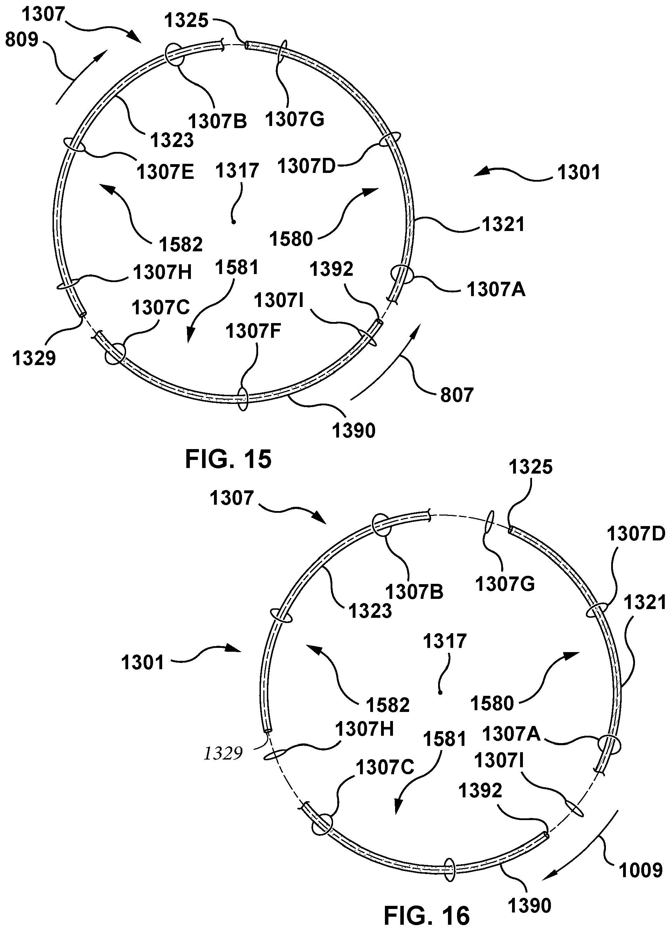

FIG. 15 illustrates an end view of the first coil assembly 1301 similar to FIG. 9, in which the inflow end attachment members 1307 are received on one or more of the first helical coil 1321, the second helical coil 1323, or the third helical coil 1390. In the depicted embodiment, the transcatheter heart valve prosthesis includes a total of nine inflow end attachment members 1307. For sake of illustration, the nine inflow end attachment members 1307 are referred to herein as first attachment member 1307A, second attachment member 1307B, third attachment member 1307C, fourth attachment member 1307D, fifth attachment member 1307E, sixth attachment member 1307F, seventh attachment member 1307G, eighth attachment member 1307H, and ninth attachment member 1307I.

The first helical coil 1321 can receive a first number 1580 of attachment members of the inflow end attachment members 1307, the second helical coil 1323 can receive a second number 1582 of attachment members of the inflow end attachment members 1307, and the third helical coil 1390 can receive a third number 1581 of attachment members of the inflow end attachment members 1307. In aspects, the first number 1580, the second number 1582, and the third number 1581 can be the same, for example, with the first number 1580, the second number 1582, and the third number 1581 each comprising three attachment members. However, in other aspects, one or more of the first number 1580, the second number 1582, or the third number 1581 of attachment members may be different. In aspects, the first number 1580, the second number 1582, and the third number 1581 of attachment members may each be attached to about one third of the valve prosthesis 10.

As illustrated in FIG. 15, the first helical end 1325, the second helical end 1329, and the third helical end 1392 may be spaced apart within a range from about 90 degrees to about 150 degrees, or about 120 degrees. The helical ends 1325, 1329, 1392 may be located at substantially the same axial location along the coil axis 1317, such that a plane that is perpendicular to the coil axis 1317 can intersect the helical ends 1325, 1329, 1392. The first helical coil 1321 receives the seventh attachment member 1307G, the second helical coil 1323 receives the eighth attachment member 1307H, and the third helical coil 1390 receives the ninth attachment member 1307I. In this way, the seventh attachment member 1307G, the eighth attachment member 1307H, and the ninth attachment member 1307I may be spaced circumferentially apart within a range from about 90 degrees to about 150 degrees, or about 120 degrees. FIG. 15 illustrates the first coil assembly 1301 after the first coil assembly 1301 has been rotated in the first rotational direction 807 (e.g., also illustrated in FIG. 14) to receive the inflow end attachment members 1307. In this way, the final attachment member to be received by the first helical coil 1321 is the seventh attachment member 1307G, the final attachment member to be received by the second helical coil 1323 is the eighth attachment member 1307H, and the final attachment member to be received by the third helical coil 1390 is the ninth attachment member 1307I. After the first coil assembly 1301 receives the inflow end attachment members 1307, and, likewise, after the second coil assembly 1303 receives the outflow end attachment members 1309, the valve prosthesis 1310 may be in the radially-collapsed position (e.g., illustrated in FIGS. 19-20).

FIG. 16 illustrates the first coil assembly 1301 after the first coil assembly 1301 has been rotated in the second rotational direction 1009 that is opposite the first rotational direction 807. For example, the first helical coil 1321, the second helical coil 1323, and the third helical coil 1390 can be rotated in the second rotational direction 1009 that is opposite the first rotational direction 807. By rotating the first coil assembly 1301 in the second rotational direction 1009, the first coil assembly 1301 can release at least some of the inflow end attachment members 1307. For example, methods comprise simultaneously rotating the first helical coil 1321, the second helical coil 1323, and the third helical coil 1390 to release the seventh attachment member 1307G from the first helical coil 1321, the eighth attachment member 1307H from the second helical coil 1323, and the ninth attachment member 1307I from the third helical coil 1390.

In aspects, the seventh attachment member 1307G, the eighth attachment member 1307H, and the ninth attachment member 1307I can be released simultaneously. For example, due to the seventh attachment member 1307G, the eighth attachment member 1307H, and the ninth attachment member 1307I being spaced circumferentially apart about 120 degrees, due to the helical ends 1325, 1329, 1392 being spaced circumferentially apart about 120 degrees, and due to the helical coils 1321, 1323, 1390 being rotated simultaneously, the seventh attachment member 1307G, the eighth attachment member 1307H, and the ninth attachment member 1307I can be removed from the helical coils 1321, 1323, 1390 at substantially the same time. The first helical end 1325 may pass through the seventh attachment member 1307G at substantially the same time that the second helical end 1329 passes through the eighth attachment member 1307H and at substantially the same time that the third helical end 1392 passes through the ninth attachment member 1307I.

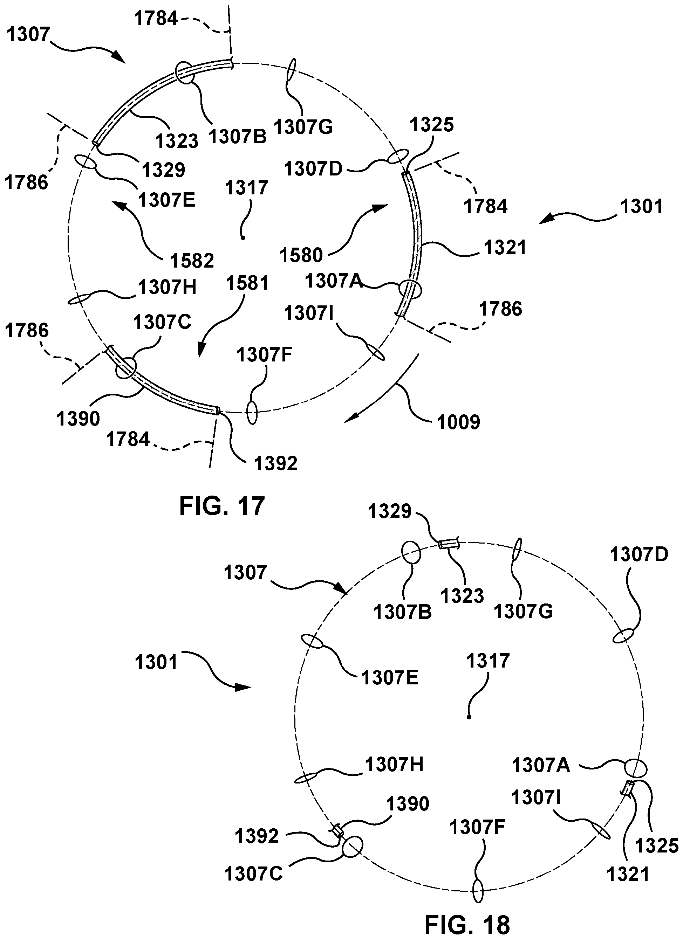

FIG. 17 illustrates the first coil assembly 1301 continuing to rotate in the second rotational direction 1009 following the position of FIG. 16. As the first coil assembly 1301 rotates, additional attachment members can be released. For example, the first helical coil 1321 can be rotated to release fourth attachment member 1307D, the second helical coil 1323 can be rotated to release the fifth attachment member 1307E, and the third helical coil 1390 can be rotated to release a sixth attachment member 1307F. In aspects, the fourth attachment member 1307D, the fifth attachment member 1307E, and the sixth attachment member 1307F can be released simultaneously due to the helical ends 1325, 1329, 1392 exiting the attachment members 1307D, 1307E, 1307F at substantially the same time. FIG. 17 illustrates the plurality of rotational positions 1784, 1786 at which the first coil assembly 1301 can release the inflow end attachment members 1307. For example, when the helical ends 1325, 1329, 1392 reach the first rotational position 1784, the set or trio of attachment members 1307D, 1307E, 1307F have been released. When the helical ends 1325, 1329, 1392 reach the second rotational position 1786, another set or trio of attachment members 1307A, 1307B, 1307C have been simultaneously released. As such, as illustrated in FIG. 18, when the helical ends 1325, 1329, 1392 reach the second rotational position 1786, all of the attachment members have been released.

Referring to FIGS. 19-21, methods can comprise percutaneously delivering a transcatheter heart valve prosthesis in a radially-collapsed position to a treatment site. FIGS. 19-21 schematically illustrate a first coil assembly 1901 and a second coil assembly 1903 attached to a transcatheter heart valve prosthesis 1910. Although the inner and intermediate shafts of the delivery system are not shown for sake of clarity, the first coil assembly 1901 is disposed over and attached to the intermediate shaft 320 of the delivery system and the second coil assembly 1903 is disposed over and attached to the inner shaft 314 of the delivery system 300. The transcatheter heart valve prosthesis 1910 is shown with a plurality of inflow end attachment members 1907 and a plurality of outflow end attachment members 1909. The transcatheter heart valve prosthesis 1910 may be similar to the transcatheter heart valve prostheses 10, 210 described above. The first coil assembly 1901 may include either the first coil assembly 701 comprising two helical coils illustrated in FIGS. 7-12 or the first coil assembly 1301 comprising three helical coils illustrated in FIGS. 13-18. Similarly, the second coil assembly 1903 may include either the second coil assembly 703 comprising two helical coils illustrated in FIGS. 7-12 or the second coil assembly 1303 comprising three helical coils illustrated in FIGS. 13-18. The first coil assembly 1901 is attached to an inflow end 1948 of the transcatheter heart valve prosthesis 1910 and the second coil assembly 1903 is attached to an outflow end 1949 of the transcatheter heart valve prosthesis 1910 such that the transcatheter heart valve prosthesis 1910 can be in the radially-collapsed position. In this way, the coil assemblies 1901, 1903 at the inflow end 1948 and the outflow end 1949 may be identical (e.g., comprising either two helical coils or three helical coils), or, alternatively, the coil assembly at one end of the transcatheter heart valve prosthesis 1910 may be different than the coil assembly at the opposite end of the transcatheter heart valve prosthesis 1910, for example, by comprising two helical coils versus three helical coils. While in the radially-collapsed position, transcatheter heart valve prosthesis 1910 can be delivered to the treatment site. To facilitate delivery, in aspects, the retractable sheath 326 of the delivery system 300 covers the transcatheter heart valve prosthesis 1910, the first coil assembly 1901, and the second coil assembly 1903. In this way, during delivery, the retractable sheath 326 circumferentially surrounds the transcatheter heart valve prosthesis 1910, the first coil assembly 1901, and the second coil assembly 1903 as the transcatheter heart valve prosthesis 1910 is percutaneously delivered to the treatment site.

Referring to FIG. 20, upon reaching the treatment site, the retractable sheath 326 is retracted, for example, by being moved in a proximal direction 2098. After moving in the proximal direction 2098, the retractable sheath 326 may no longer circumferentially surround or cover the valve prosthesis 1910. In an embodiment, as depicted in FIG. 20, a degree of radial expansion of a midportion of the valve prosthesis 1910 may occur upon retraction of the retractable sheath 326 while the ends thereof are still restrained by the first coil assembly 1901 and the second coil assembly 1903. The midportion of the valve prosthesis 1910 does not expand to its full radially expanded configuration, since the ends thereof are still restrained by the first coil assembly 1901 and the second coil assembly 1903. In another embodiment, the system may be configured such that no radial expansion of a midportion of the valve prosthesis 1910 occurs upon retraction of the retractable sheath 326 while the ends thereof are still restrained by the first coil assembly 1901 and the second coil assembly 1903. Rather, the system may be configured such that the first coil assembly 1901 and the second coil assembly 1903 keep the valve prosthesis 1910 in its radially compressed configuration even after the retracted sheath 326 is retracted to help avoid undesired anatomical interaction or to further enable recapture.

Referring to FIG. 21, after retracting the retractable sheath 326, the first coil assembly 1901 and the second coil assembly 1903 can be detached from the transcatheter heart valve prosthesis 1910. For example, the first coil assembly 1901 can be detached from the inflow end attachment members 1907 in the manner described relative to FIGS. 7-18. Likewise, the second coil assembly 1903 can be detached from the outflow end attachment members 1909 in the manner described relative to FIGS. 7-18. Accordingly, upon detaching from the delivery system 300, the transcatheter heart valve prosthesis 1910 can move from the radially-collapsed position to the radially-expanded position. Due to the simultaneous release of pairs or trios of attachment members by the first coil assembly 1901 and/or the second coil assembly 1903, the likelihood of the transcatheter heart valve prosthesis 1910 inadvertently moving in an unwanted direction upon deployment may be reduced. Another potential advantage of the coil assemblies 1901, 1903 is that the likelihood of backfolding of crowns of the transcatheter heart valve prosthesis 1910 during deployment may be reduced, for example, due to the aforementioned method of detaching the transcatheter heart valve prosthesis 1910 from the coil assemblies 1901, 1903. In addition, in aspects, a central or middle region of the transcatheter heart valve prosthesis 1910 can optionally be radially-expanded while the inflow and outflow ends 1948, 1949 are constrained by the coil assemblies 1901, 1903 thus allowing for re-positioning or recapture of the transcatheter heart valve prosthesis 1910 if desired. In this way, a more controlled expansion and release of the transcatheter heart valve prosthesis 1910 can be achieved.

Simultaneous release of pairs or trios of attachment members by the first coil assembly 1901 and the second coil assembly 1903 may also reduce risk of backfolding of crowns that may otherwise occur with non-simultaneous or non-symmetrical release. For example, without intending to be bound by theory, non-simultaneous or non-symmetrical release may allow one or two offset (e.g., radially asymmetric) crowns to expand, contact the anatomy, and displace the frame in an unintended or uncontrollable manner or may cause the one or two offset crows to backfold based on a lack of opposing radially-symmetric force. In addition, in some embodiments, the middle section of the prosthesis 1910 may be allowed to expand prior to release of the attachment members by the first coil assembly 1901 and the second coil assembly 1903. In this way, the expanded middle section of the valve may fix within the anatomy of the patient, thereby holding the prosthesis 1910 stable at which point the release of the attachment members by the first coil assembly 1901 and the second coil assembly 1903 can occur with the stable middle section anchoring the prosthesis 1910 during crown release. Similarly, by allowing the middle section of the prosthesis 1910 to expand prior to release of the attachment members by the first coil assembly 1901 and the second coil assembly 1903, a user (e.g., physician) can evaluate the placement (e.g., depth) of the prosthesis 1910 prior to full deployment. If the user is pleased with the location and placement of the valve within the patient's anatomy, the user may then deploy the prosthesis 1910 via release of the attachment members by the first coil assembly 1901 and the second coil assembly 1903. Alternatively, if the user determines that the placement of the prosthesis 1910 after expansion of the middle section is unsatisfactory, the user may recapture (e.g., recollapse) the prosthesis 1910 by distally advancing the retractable sheath 326, reposition the prosthesis 1910, and then expand and deploy the prosthesis 1910 in accordance with the methods discussed above.

It should be understood that while various aspects have been described in detail relative to certain illustrative and specific examples thereof, the present disclosure should not be considered limited to such, as numerous modifications and combinations of the disclosed features are possible without departing from the scope of the following claims.

The Following examples are illustrative of the techniques described herein.

Example 1: A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising: a coil assembly extending along a coil axis and configured to be releasably attached to the plurality of attachment members, the coil assembly comprising: a first helical coil wound around and extending along the coil axis, the first helical coil terminating at a first helical end and configured to receive a first attachment member of the plurality of attachment members; and a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members.

Example 2: The delivery system of claim 1, wherein the first helical end and the second helical end are spaced circumferentially apart within a range from about 150 degrees to about 210 degrees.

Example 3: The delivery system of claim 2, wherein the first attachment member and the second attachment member are spaced circumferentially apart within a range from about 150 degrees to about 210 degrees, and wherein the first attachment member and the second attachment member are attached to the inflow end.

Example 4: The delivery system of claim 1, wherein the first helical coil is configured to receive a first number of attachment members of the plurality of attachment members and the second helical coil is configured to receive a second number of attachment members of the plurality of attachment members, and wherein the first number of attachment members is different than the second number of attachment members.

Example 5: The delivery system of claim 4, wherein the first number of attachment members are attached to a first circumferential half of the heart valve prosthesis and the second number of attachment members are attached to a second circumferential half of the heart valve prosthesis.

Example 6: The delivery system of claim 1, further comprising a second coil assembly extending along a coil axis and configured to be releasably attached to the plurality of attachment members, the second coil assembly comprising: a first helical coil wound around and extending along the coil axis, the first helical coil terminating at a first helical end and configured to receive a third attachment member of the plurality of attachment members; and a second helical coil wound around and extending along the coil axis, the second helical coil coaxial and intertwined with the first helical coil, the second helical coil terminating at a second helical end that is axially aligned with the first helical end, the second helical coil configured to receive a fourth attachment member of the plurality of attachment members.

Example 7: The delivery system of claim 6, wherein the third attachment member and the fourth attachment member are attached to the outflow end such that the second coil assembly is attached to the outflow end.

Example 8: A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising: a coil assembly extending along a coil axis and configured to be releasably attached to the plurality of attachment members, the coil assembly comprising: a first helical coil wound around and extending along the coil axis, the first helical coil terminating at a first helical end and configured to receive a first attachment member of the plurality of attachment members; a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members; and a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a third attachment member of the plurality of attachment members.

Example 9: The delivery system of claim 8, wherein the first helical end, the second helical end, and the third helical end are spaced apart within a range from about 90 degrees to about 150 degrees.

Example 10: The delivery system of claim 8, wherein the first attachment member, the second attachment member, and the third attachment member are spaced circumferentially apart within a range from about 90 degrees to about 150 degrees, and wherein the first attachment member, the second attachment member, and the third attachment member are attached to the inflow end.

Example 11: The delivery system of claim 8, wherein the first helical coil is configured to receive a first number of attachment members of the plurality of attachment members, the second helical coil is configured to receive a second number of attachment members of the plurality of attachment members, and the third helical coil is configured to receive a third number of attachment members of the plurality of attachment members, and wherein the first number of attachment members, the second number of attachment members, and the third number of attachment members are the same.

Example 12: The delivery system of claim 8, further comprising a second coil assembly extending along a coil axis and configured to be releasably attached to the plurality of attachment members, the second coil assembly comprising:
a first helical coil wound around and extending along the coil axis, the first helical coil terminating at a first helical end and configured to receive a fourth attachment member of the plurality of attachment members; a second helical coil wound around and extending along the coil axis, the second helical coil coaxial and intertwined with the first helical coil, the second helical coil terminating at a second helical end that is axially aligned with the first helical end, the second helical coil configured to receive a fifth attachment member of the plurality of attachment members; and a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a sixth attachment member of the plurality of attachment members.

Example 13: The delivery system of claim 12, wherein the fourth attachment member, the fifth attachment member, and the sixth attachment member are attached to the outflow end.

Example 14: A method of deploying a heart valve prosthesis at a treatment site within a patient, the heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the method comprising: receiving a first attachment member of the plurality of attachment members on a first helical coil, the first helical coil wound around and extending along a coil axis; receiving a second attachment member of the plurality of attachment members on a second helical coil, the second helical coil coaxial and intertwined with the first helical coil; percutaneously delivering the heart valve prosthesis in the radially-collapsed position to the treatment site; and simultaneously rotating the first helical coil and the second helical coil to release the first attachment member from the first helical coil and the second attachment member from the second helical coil and cause the heart valve prosthesis to move from the radially-collapsed position to the radially-expanded position.

Example 15: The method of claim 14, wherein the first attachment member and the second attachment member are released simultaneously.

Example 16: The method of claim 15, wherein the first helical coil terminates at a first helical end and the second helical coil terminates at a second helical end, and wherein the first helical end and the second helical end are spaced circumferentially apart within a range from about 150 degrees to about 210 degrees.

Example 17: The method of claim 16, wherein the first attachment member and the second attachment member are attached to the inflow end.

Example 18: The method of claim 14, wherein receiving the first attachment member further comprises receiving a first number of attachment members of the plurality of attachment members on the first helical coil, and wherein receiving the second attachment member further comprises receiving a second number of attachment members of the plurality of attachment members on the second helical coil, and wherein the first number of attachment members is different than the second number of attachment members.

Example 19: The method of claim 14, further comprising receiving a third attachment member of the plurality of attachment members on a third helical coil, the third helical coil coaxial and intertwined with the first helical coil and the second helical coil.

Example 20: The method of claim 19, wherein the simultaneously rotating further comprises simultaneously rotating the first helical coil, the second helical coil, and the third helical coil to release the first attachment member, the second attachment member, and the third attachment member.

Example 21. The method of claim 14, wherein the frame of the heart valve prosthesis includes a plurality of sinusoidal rings, each ring being sutured to an adjacent ring via abutting crowns.

Further disclosed herein is the subject-matter of the following clauses:
1. A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising:
   a coil assembly extending along a coil axis and configured to be releasably attached to the plurality of attachment members, the coil assembly comprising:
   a first helical coil wound around and extending along the coil axis, the first helical coil terminating at a first helical end and configured to receive a first attachment member of the plurality of attachment members; and
   a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members.
2. The delivery system of clause 1, wherein the first helical end and the second helical end are spaced circumferentially apart within a range from about 150 degrees to about 210 degrees.
3. The delivery system of clause 2, wherein the first attachment member and the second attachment member are spaced circumferentially apart within a range from about 150 degrees to about 210 degrees, and wherein the first attachment member and the second attachment member are attached to the inflow end.
4. The delivery system of clause 1 or of any of the preceding clauses, wherein the first helical coil is configured to receive a first number of attachment members of the plurality of attachment members and the second helical coil is configured to receive a second number of attachment members of the plurality of attachment members, and wherein the first number of attachment members is different than the second number of attachment members.
5. The delivery system of clause 4, wherein the first number of attachment members are attached to a first circumferential half of the heart valve prosthesis and the second number of attachment members are attached to a second circumferential half of the heart valve prosthesis.
6. The delivery system of clause 1 or of any of the preceding clauses, further comprising a second coil assembly extending along a coil axis and configured to be releasably attached to the plurality of attachment members, the second coil assembly comprising:
   a first helical coil wound around and extending along the coil axis, the first helical coil terminating at a first helical end and configured to receive a third attachment member of the plurality of attachment members; and
   a second helical coil wound around and extending along the coil axis, the second helical coil coaxial and intertwined with the first helical coil, the second helical coil terminating at a second helical end that is axially aligned with the first helical end, the second helical coil configured to receive a fourth attachment member of the plurality of attachment members.
7. The delivery system of clause 6, wherein the third attachment member and the fourth attachment member are attached to the outflow end such that the second coil assembly is attached to the outflow end.
8. The delivery system of clause 1 or of any of the preceding clauses, the delivery system further comprising the heart valve prosthesis, wherein the frame of the heart valve prosthesis includes a plurality of sinusoidal rings, each ring being sutured to an adjacent ring via abutting crowns.
9. A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising:
   a coil assembly extending along a coil axis and configured to be releasably attached to the plurality of attachment members, the coil assembly comprising:
   a first helical coil wound around and extending along the coil axis, the first helical coil terminating at a first helical end and configured to receive a first attachment member of the plurality of attachment members;
   a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members; and
   a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a third attachment member of the plurality of attachment members.
10. The delivery system of clause 9, wherein the first helical end, the second helical end, and the third helical end are spaced apart within a range from about 90 degrees to about 150 degrees.
11. The delivery system of clause 9 or of any of clauses 9-10, wherein the first attachment member, the second attachment member, and the third attachment member are spaced circumferentially apart within a range from about 90 degrees to about 150 degrees, and wherein the first attachment member, the second attachment member, and the third attachment member are attached to the inflow end.
12. The delivery system of clause 9 or of any of clauses 9-11, wherein the first helical coil is configured to receive a first number of attachment members of the plurality of attachment members, the second helical coil is configured to receive a second number of attachment members of the plurality of attachment members, and the third helical coil is configured to receive a third number of attachment members of the plurality of attachment members, and wherein the first number of attachment members, the second number of attachment members, and the third number of attachment members are the same.
13. The delivery system of clause 9 or of any of clauses 9-12, further comprising a second coil assembly extending along a coil axis and configured to be releasably attached to the plurality of attachment members, the second coil assembly comprising:
   a first helical coil wound around and extending along the coil axis, the first helical coil terminating at a first helical end and configured to receive a fourth attachment member of the plurality of attachment members;
   a second helical coil wound around and extending along the coil axis, the second helical coil coaxial and intertwined with the first helical coil, the second helical coil terminating at a second helical end that is axially aligned with the first helical end, the second helical coil configured to receive a fifth attachment member of the plurality of attachment members; and
   a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a sixth attachment member of the plurality of attachment members.
14. The delivery system of clause 13, wherein the fourth attachment member, the fifth attachment member, and the sixth attachment member are attached to the outflow end.
15. The delivery system of clause 9 or of any of clauses 9-14, the delivery system further comprising the heart valve prosthesis, wherein the frame of the heart valve prosthesis includes a plurality of sinusoidal rings, each ring being sutured to an adjacent ring via abutting crowns.

## Claims

1. A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising:
a coil assembly extending along a coil axis and configured to be releasably attached to the plurality of attachment members, the coil assembly comprising:
a first helical coil wound around and extending along the coil axis, the first helical coil terminating at a first helical end and configured to receive a first attachment member of the plurality of attachment members; and
a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members.

2. The delivery system of claim 1, wherein the first helical end and the second helical end are spaced circumferentially apart within a range from about 150 degrees to about 210 degrees.

3. The delivery system of claim 2, wherein the first attachment member and the second attachment member are spaced circumferentially apart within a range from about 150 degrees to about 210 degrees, and wherein the first attachment member and the second attachment member are attached to the inflow end.

4. The delivery system of any of the preceding claims, wherein the first helical coil is configured to receive a first number of attachment members of the plurality of attachment members and the second helical coil is configured to receive a second number of attachment members of the plurality of attachment members, and wherein the first number of attachment members is different than the second number of attachment members.

5. The delivery system of claim 4, wherein the first number of attachment members are attached to a first circumferential half of the heart valve prosthesis and the second number of attachment members are attached to a second circumferential half of the heart valve prosthesis.

6. The delivery system of any of the preceding claims, further comprising a second coil assembly extending along a coil axis and configured to be releasably attached to the plurality of attachment members, the second coil assembly comprising:
a first helical coil wound around and extending along the coil axis, the first helical coil terminating at a first helical end and configured to receive a third attachment member of the plurality of attachment members; and
a second helical coil wound around and extending along the coil axis, the second helical coil coaxial and intertwined with the first helical coil, the second helical coil terminating at a second helical end that is axially aligned with the first helical end, the second helical coil configured to receive a fourth attachment member of the plurality of attachment members.

7. The delivery system of claim 6, wherein the third attachment member and the fourth attachment member are attached to the outflow end such that the second coil assembly is attached to the outflow end.

8. The delivery system of any of the preceding claims, the delivery system further comprising the heart valve prosthesis, wherein the frame of the heart valve prosthesis includes a plurality of sinusoidal rings, each ring being sutured to an adjacent ring via abutting crowns.

9. A delivery system for delivering a heart valve prosthesis comprising a frame and a prosthetic valve component comprising at least one leaflet attached to the frame, the frame extending along a longitudinal axis between an inflow end and an outflow end, the frame comprising a plurality of struts and being configured to transition between a radially-collapsed position and a radially-expanded position, the frame comprising a plurality of attachment members at one or more of the inflow end and the outflow end, the delivery system comprising:
a coil assembly extending along a coil axis and configured to be releasably attached to the plurality of attachment members, the coil assembly comprising:
a first helical coil wound around and extending along the coil axis, the first helical coil terminating at a first helical end and configured to receive a first attachment member of the plurality of attachment members;
a second helical coil wound around and extending along the coil axis, the second helical coil coaxial and intertwined with the first helical coil, the second helical coil terminating at a second helical end that is axially aligned with the first helical end, the second helical coil configured to receive a second attachment member of the plurality of attachment members; and
a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a third attachment member of the plurality of attachment members.

10. The delivery system of claim 9, wherein the first helical end, the second helical end, and the third helical end are spaced apart within a range from about 90 degrees to about 150 degrees.

11. The delivery system of any of claims 9-10, wherein the first attachment member, the second attachment member, and the third attachment member are spaced circumferentially apart within a range from about 90 degrees to about 150 degrees, and wherein the first attachment member, the second attachment member, and the third attachment member are attached to the inflow end.

12. The delivery system of any of claims 9-11, wherein the first helical coil is configured to receive a first number of attachment members of the plurality of attachment members, the second helical coil is configured to receive a second number of attachment members of the plurality of attachment members, and the third helical coil is configured to receive a third number of attachment members of the plurality of attachment members, and wherein the first number of attachment members, the second number of attachment members, and the third number of attachment members are the same.

13. The delivery system of any of claims 9-12, further comprising a second coil assembly extending along a coil axis and configured to be releasably attached to the plurality of attachment members, the second coil assembly comprising:
a first helical coil wound around and extending along the coil axis, the first helical coil terminating at a first helical end and configured to receive a fourth attachment member of the plurality of attachment members;
a second helical coil wound around and extending along the coil axis, the second helical coil coaxial and intertwined with the first helical coil, the second helical coil terminating at a second helical end that is axially aligned with the first helical end, the second helical coil configured to receive a fifth attachment member of the plurality of attachment members; and
a third helical coil wound around and extending along the coil axis, the third helical coil coaxial and intertwined with the first helical coil and the second helical coil, the third helical coil terminating at a third helical end that is axially aligned with the first helical end and the second helical end, the third helical coil configured to receive a sixth attachment member of the plurality of attachment members.

14. The delivery system of claim 13, wherein the fourth attachment member, the fifth attachment member, and the sixth attachment member are attached to the outflow end.

15. The delivery system of any of claims 9-14, the delivery system further comprising the heart valve prosthesis, wherein the frame of the heart valve prosthesis includes a plurality of sinusoidal rings, each ring being sutured to an adjacent ring via abutting crowns.
